# EUROPEAN PATENT APPLICATION

(11) **EP 2 669 683 A1**
(43) Date of publication of application: **04.12.2013**
(21) Application number: 12739322.1
(22) Date of filing: 26.01.2012
(51) Int. Cl.: G01N 33/579, C12M 1/34, C12Q 1/37

(54) **METHOD AND APPARATUS FOR MEASURING PHYSIOLOGICALLY ACTIVE BIOLOGICAL SUBSTANCE**

(30) Priority: 26.01.2011 JP 2011014574; 31.03.2011 JP 2011080714
(71) Applicant: Kowa Company Ltd., Naka-ku Nagoya-shi Aichi 460-8625 (JP)
(72) Inventor: INADA, Katsuya, Morioka-shi Iwate 020-0106 (JP); ENDO, Shigeatsu, Morioka-shi Iwate 020-8505 (JP); HIRONO, Taisuke, Hamamatsu-shi Shizuoka 431-2103 (JP); ASANO, Takaharu, Hamamatsu-shi Shizuoka 431-2103 (JP)
(74) Representative: Elsy, David
(86) International application number: PCT/JP2012/051715
(87) International publication number: WO 2012/102353

(57) **Abstract**

The purpose of the present invention is to improve the measurement accuracy in the measurement of the concentration of a physiologically active biological substance in a sample by a stirring turbidimetry, a light scattering method or an AL-bound beads method, wherein the purpose can be achieved by preventing the occurrence of aggregation or gelation that is caused by the stirring of a mixed solution and is not associated with the physiologically active substance. AL is mixed with a sample containing a physiologically active biological substance, and the aggregation of a protein which is associated with the reaction between AL and the physiologically active substance in the mixed solution is detected while stirring the mixed solution, wherein the occurrence of the aggregation or gelation of a protein which is not associated with the reaction between AL and the physiologically active substance in the mixed solution can be prevented by adding a specific protein that has been heated in advance and/or a specific surfactant to the mixed solution.

## Description

### Technical Field

The present invention relates to a measurement method and a measurement apparatus for detecting or measuring a concentration of a physiologically active substance derived from an organism, which has a property of gelation by a reaction with Amoebocyte lysate (hereinafter, also referred to as "AL") such as endotoxin and β-D-glucan, in a sample containing the physiologically active substance.

### Background Art

Endotoxin is a lipopolysaccharide present in a cell wall of a Gram-negative bacterium and is the most typical pyrogen. If a transfusion, a medicine for injection, or the blood contaminated with the endotoxin is introduced into the human body, the endotoxin may induce severe side effects such as fever and shock. Therefore, it has been required that the above mentioned medicine or the like be kept so as not to be contaminated with endotoxin. On the other hand, measurement of endotoxin in the blood of a sepsis patient also contributes to prevention or treatment of severe endotoxin shock.

As a semi-quantitative test method for detecting endotoxin contamination of a medicine for injection and the like, the following method was previously conducted. That is, to the ear vein of a healthy rabbit having 1.5 kg or more of the body weight, a sample heated to 37°C is injected, and the body temperature is measured at an interval of 30 minutes or less to 3 hours after the injection. The average value of the body temperatures of the rabbit measured twice at an interval of 30 minutes during the time from 40 minutes before the injection to the injection, is taken as the body temperature of the subject, and the difference between the body temperature of the subject and the maximum body temperature is taken as the increase of the body temperature.

Three rabbits are used in measuring the increase of the body temperature, and the following determination is performed with the sum of the body temperature increases of the three rabbits. First, in the case where the sum of the body temperature increases of the three rabbits is 1.3°C or lower, it is determined as pyrogen negative, and in the case where the total of the body temperature increases is 2.5°C or higher, it is determined as pyrogen positive. Then, in the case where the sum of the body temperature increases of the three rabbits is greater than 1.3°C and less than 2.5°C, a test by further three rabbits is added. In the case where the sum of the body temperature increases of the total six rabbits is 3.0°C or lower, it is determined as pyrogen negative, and in the case where the sum of the body temperature increases of the total six rabbits is 4.2°C or higher, it is determined as pyrogen positive. In the case where the sum of the temperature increases at the time is greater than 3.0°C and less than 4.2°C, a test for further three rabbits is added. In the case where the body temperature increase in the total 9 rabbits is less than 5.0°C, it is determined as pyrogen negative, and in the case where the body temperature increase in the total 9 rabbits is 5.0°C or higher, it is determined as pyrogen positive.

Further, β-D-glucan is a polysaccharide which constitutes a cell membrane that is characteristic of a fungus. Measurement of β-D-glucan is effective, for example, for screening infectious diseases due to a variety of fungi including not only fungi which are frequently observed in general clinical practices such as Candida, Aspergillus, and Cryptococcus, but also rare fungi.

By the way, AL contains serine proteases which are activated by endotoxin, β-D-glucan and the like. Then, whenAL reacts with endotoxin or β-D-glucan, a coagulogen present in AL is hydrolyzed into a coagulin by an enzyme cascade of the serine proteases activated depending on the amount of endotoxin or β-D-glucan, and the coagulin is associated with one another to form an insoluble gel. By using this property of AL, endotoxin or β-D-glucan can be detected with a high sensitivity. In recent years, a method using this was invented, in which AL is used in detection or measurement of the concentration of endotoxin and the like.

As a method for detecting a physiologically active substance derived from an organism (hereinafter, also referred to as a predetermined physiologically active substance) which can be detected by AL, such as endotoxin or β-D-glucan, or measuring the concentration thereof, there is a semi-quantitative gelation method, which comprises: standing a mixture obtained by mixing a sample to be used for detection or concentration-measurement of the predetermined physiologically active substance (hereinafter, also simply referred to as "measurement of the predetermined physiologically active substance") with a reagent prepared to be based on AL (AL reagent); inverting the container after a lapse of a predetermined time period; and judging whether gelation has occurred or not based on the presence or absence of falling down of the sample to examine whether or not the sample contains endotoxin at a certain concentration or higher.

As another example of the method, there is given a turbidimetric method, which comprises standing the mixture obtained by mixing a sample to be used for measurement of the predetermined physiologically active substance with AL at 37°C being maintained, and analyzing the sample by measuring the time course of the turbidity of the sample caused by gel formation by a reaction between AL and the predetermined physiologically active substance. In this turbidimetric method, the time point when the transmittance is lowered to a certain value or lower after initiation of the measurement, is taken as the gelation time. The quantification utilizes the fact that the gelation time correlates with the endotoxin amount in the analyte. That is, the endotoxin amount in the analyte is calculated from a calibration curve, which is previously prepared by measuring plural standard samples containing known amounts of endotoxin mixed in, and the gelation time obtained by the measurement.

In the case where measurement of the predetermined physiologically active substance is conducted by the turbidimetric method, a mixture of the sample to be measured and the AL is formed in a dry-heat-sterilized glass measurement cell. Then, the mixture is left to stand still, and the gelation is optically measured from the outside. On the other hand, there is also the stirring turbidimetric method in which the gelation is measured with the turbidimetric method while stirring the analyte mixed with the AL reagent. In this stirring turbidimetric method, the sample is stirred as well during the measurement by rotation of the stirring bar introduced into the glass cell for measurement. Then, similarly to the turbidimetric method, the endotoxin amount in the analyte is calculated by the calibration curve method. With the stirring turbidimetric method, it is possible to perform faster and more stable measurement than the turbidimetric method by stirring the sample during the measurement.

Further, there is also the AL-bound bead method in which a mixture containing particulates in which AL is bound onto the surface (hereinafter, also referred to as the AL-bound bead) as well as the sample to be measured and the AL, is formed, and the gelation is measured with the stirring turbidimetric method. With this AL-bound bead method, the time course is measured and analyzed of the turbidity of the sample caused by aggregation of the AL-bound bead formed by the reaction of the sample, AL and the AL-bound bead. In this AL-bound bead method, the time point when the transmittance is lowered to a certain value or lower after initiation of the measurement, is taken as the gelation time. Then, the endotoxin amount in the analyte is calculated by the calibration curve method similarly to the stirring turbidimetric method. The AL-bound bead method allows faster measurement than the turbidimetric method or the stirring turbidimetric method. This is mainly because the AL-bound bead aggregates greater than the coagulin by 10 to 100 or more folds and thus the change of the transmittance becomes sharp in the AL-bound bead method.

Furthermore, there is also proposed the method for counting particle by laser light scattering, which comprises stirring a mixture of a sample to be measured and the AL using, for example, a magnetic stirring bar to form fine gel-particles, and determining the presence of the predetermined physiologically active substance in the sample in a short period of time based on the intensity of laser light scattered by the gel-particles (hereinafter, also simply referred to as the light scattering method). If gel particles are formed by the reaction of the sample with AL, and they aggregate to each other when the reaction proceeds, a spike-shape peak signal is detected in the scattered light. The time point when these peak signals are detected in a certain or higher frequency is taken as the aggregation initiation time in the light scattering method. Similarly to the gelation time, the aggregation initiation time correlates with the endotoxin amount in the analyte, and thus the endotoxin amount in the analyte is calculated by the calibration curve method using this.

Also in the light scattering method, the sample is stirred during the measurement by rotation of a stirring bar introduced into the glass sample cell, but the object of the stirring is different from that during the stirring turbidimetric method. The aforementioned peak signal is detected when the gel particle passes across the laser light emitted into the analyte. The gel particle is moved by the stirring so that the gel particle can pass across the laser light. The light scattering method allows faster and high-sensitive measurement than the turbidimetric method or stirring turbidimetric method. This is a factor to the fact that the light scattering method allows detection of the gelation mainly at the initial stage of the gelation, that is to say, at the time point when a small gel particle is formed.

On the other hand, there is a method in which a synthetic substrate for a clotting enzyme added to a reagent is previously introduced, and the synthetic substrate decomposed by the clotting enzyme is measured by a phenomenon of coloring or fluorescence, and further luminescence. Herein, a method using the coloring is called the colorimetric method, which is widely used as one of important methods for measuring a predetermined physiologically active substance. Examples of the colorimetric method includes the end point-colorimetric method using the correlation between the concentration of the predetermined physiologically active substance and the release amount of a chromophore after a certain reaction time; and the kinetic-colorimetric method using the correlation between the concentration of the predetermined physiologically active substance and the time needed for the absorbance or transmittance of the mixture to reach a certain value, or temporal change rate of the coloring.

Herein, in the case where the mixture of the AL reagent and the sample is stirred with a stirring bar in a glass sample cell such as during the stirring turbidimetric method or light scattering method, there is a problem that the shape of the reaction curve (transmittance/absorbance (stirring turbidimetric method) or the number of the gel particles (light scatteringmethod) with respect to time) easily changes, and the accuracy of the gelation time or the aggregation initiation time obtained by the measurement is lowered (for example, see Non-Patent Literature 1). In order to avoid these problems, an algorithm to determine the gelation time or the aggregation initiation time is elaborated in each measurement method, so that somewhat change of the reaction curve has no influence on the measurement results (see, for example, Patent Literature 1).

However, particularly, for example, in the case where endotoxin in dilute plasma derivatives or water for injection is measured, the workarounds described above do not completely function, and false results may be obtained. The situation is that the causes for change of the shape of the reaction curve by the stirring cannot be specified, and there is no direct countermeasure.

### Citation List

### Patent Literature

Patent Literature 1: JP 2010-216878 A
Patent Literature 2: JP 2004-061314 A
Patent Literature 3: JP 10-293129 A
Patent Literature 4: WO 2008/038329 A
Patent Literature 5: JP 2009-150723

### Non-Patent Literature

Non-Patent Literature 1: Manabu Takahashi, Shigehiro Shibata, Yasushi Suzuki, Masahiro Kojika, Naoya Matsumoto, Tatsuyori Shozushima, Katsuya Inada, Shigeatsu Endo, "Issues in clinical application of the endotoxin measurement method using endotoxin scattering photometry", Japan journal of critical care for endotoxemia, Volume 14, No. 1, pp. 111-119, 2010

### Summary of Invention

### Technical Problem

The object of the present invention is to provide a technology for obtaining higher accuracy of the measurement in a method of measuring a physiologically active substance derived from an organism by optically measuring aggregation or gelation due to the reaction of AL, which is Amoebocyte lysate, and the physiologically active substance derived from an organism.

More specifically, the object of the present invention is to improve the accuracy of measurement for the concentration of a physiologically active substance derived from an organism in a sample by suppressing aggregation or gelation that is caused by the stirring of a mixture of the AL reagent and the sample and not derived from the physiologically active substance when the concentration is measured by the stirring turbidimetric method, the light scattering method, or the AL-bound bead method.

### Solution to Problem

The greatest characteristic of the present invention is that when aggregation or gelation of a protein is detected due to a reaction of AL with a physiologically active substance derived from an organism in a mixture obtained by mixing the AL reagent with a sample containing the physiologically active substance derived from an organism while stirring the mixture, a predetermined protein that is previously heat-treated and/or a predetermined surfactant is added to the mixture, whereby to suppress aggregation or gelation of a protein not due to the reaction of AL with the physiologically active substance derived from an organism in the mixture.

More specifically, the present invention is a method of measuring a physiologically active substance derived from an organism which is used for detection or concentration-measurement of the physiologically active substance in a sample, by detection of aggregation or gelation of a protein due to the reaction of AL, which is Amoebocyte lysate, with the physiologically active substance in a mixture produced by mixing AL and a sample containing the predetermined physiologically active substance derived from an organism while stirring the mixture, in which by adding a predetermined protein that is previously heat-treated and/or a predetermined surfactant to the mixture during the stirring, aggregation or gelation not derived from the physiologically active substance in the mixture is suppressed.

Herein, the cause for the phenomenon of the change of the reaction curve of the sample and the AL reagent by the stirring of the mixture was clearly revealed to be as follows. That is, it was found out that in the case where a sample having very small content of a physiologically active substance derived from an organism is measured, (1) proteins contained in the AL reagent and/or the sample aggregate to each other by stirring, or (2) a pro-clotting enzyme is cleaved and activated, and changes to a clotting enzyme by shear stress (physical stimulation) caused by stirring, and an aggregate not derived from a physiologically active substance derived from an organism is formed.

This aggregation not derived from a physiologically active substance derived from an organism is detected as a signal similar to that of generation of a gel particle in both methods of the stirring turbidimetric method and the light scattering method, and thus may have an influence on the shape of the reaction curve (time course), lower the measurement accuracy, and cause false measurement. On the other hand, by intensive study of the inventors, it was found out that induction of aggregation or gelation not derived from a physiologically active substance derived from an organism can be suppressed by adding a predetermined, previously heat-treated protein or predetermined surfactant at the time of mixing a sample and an AL reagent. Particularly, it was found out that activation of a clotting enzyme mainly due to stimulation of the stirring can be suppressed by adding a predetermined, previously heat-treated protein. On the other hand, it was found out that aggregation of proteins to each other contained in the AL reagent and/or sample can be suppressed by adding a surfactant. Furthermore, it was confirmed by experiments that addition of such heat-treated, predetermined protein or surfactant to the sample has no influence on measurement of aggregation or gelation derived from the physiologically active substance derived from an organism.

According to the present invention, particularly in the case where aggregation or gelation is measured under stirring in a mixture of a sample having a small content of physiologically active substance derived from an organism, and the AL reagent, it is possible to suppress aggregation or gelation caused by stirring and not due to a physiologically active substance derived from an organism. As a result thereof, it is possible to improve the accuracy in the measurement of a physiologically active substance derived from an organism.

Further, the predetermined protein that is previously heat-treated in the present invention may be at least one kind of albumin, globulin and lysozyme. It has been confirmed that induction of aggregation or gelation not derived from a physiologically active substance derived from an organism can be suppressed by adding these proteins to the mixture of the sample and the AL reagent, and thus, it is possible to suppress more certainly, aggregation or gelation caused by stirring and not due to a physiologically active substance derived from an organism. Meanwhile, as the protein that can be used in the present invention, for example, many kinds such as a protein present in the human plasma, a protein of an animal, a plant or insect can be considered to be used. For convenience of explanation, the specification is described mainly focusing on albumin.

Further, in the case where the predetermined protein that is previously heat-treated is albumin in the present invention, the final concentration of the previously heat-treated albumin when the previously heat-treated albumin is added to the mixture may be 0.015% or more and 10% or less. This can suppress more effectively aggregation or gelation caused by stirring and not due to a physiologically active substance derived from an organism. Meanwhile, the final concentration is preferably 0.03% or more and 5% or less, further preferably 0.05% or more and 0.5% or less.

Further, in the present invention, the temperature of the heat treatment that is previously performed for the predetermined protein is preferably 70°C or higher. Further, the time of the heat treatment is preferably 10 minutes or more. Further, as the heat treatment that is previously performed for the predetermined protein, autoclave sterilization) treatment may be performed, or heating treatment using equipment such as an aluminum block heater may be performed. By these heat treatments, it is possible to suppress more certainly aggregation or gelation caused by stirring and not due to a physiologically active substance derived from an organism in comparison to the case where a protein that is not heat-treated is added.

Meanwhile, the temperature of the autoclave sterilization treatment is generally 120°C or higher. With this temperature range, it is possible to suppress more certainly aggregation or gelation caused by stirring and not due to a physiologically active substancederivedfromanorganism. More specifically, the temperature of the autoclave sterilization treatment is preferably 120°C or higher and 300°C or lower, and more preferably 120°C or higher and 260°C or lower.

Further, the time of the heat treatment in the case where heat treatment is performed in the autoclave sterilization treatment, may be 10 minutes or more. With this time range, it is possible to suppress more certainly aggregation or gelation caused by stirring and not due to a physiologically active substancederivedfromanorganism. More specifically, the time of the heat treatment is preferably 10 minutes or more and 120 minutes or less. With this time range, it is possible to suppress more certainly aggregation or gelation caused by stirring and not due to a physiologically active substance derived from an organism, and inactivate more certainly a physiologically active substance derived from an organism contaminating the protein.

Further, it has been confirmed that heating of 10 minutes or more in the case where heating is performed at 95°C, which is a temperature lower than 120°C using an aluminum block heater, also has the effect of suppressing aggregation or gelation caused by stirring and not due to a physiologically active substance derived from an organism. Accordingly, in the case where the heating treatment is performed using the aluminum block heater, the temperature of the heat treatment may be 95°C or higher, more specifically 95°C or higher and 260°C or lower. Further, the heating time in the heating treatment may be 10 minutes or more, more specifically 10 minutes or more and 120 minutes or less. With adoption of the conditions of the heat treatment as described above, it is possible to suppress more certainly aggregation or gelation caused by stirring and not due to a physiologically active substance derived from an organism.

However, the heat treatment conditions in the present invention are not limited to the conditions described above if the conditions are those of the protein concentration, the heating temperature and the heating time allowing more effective suppression of aggregation or gelation caused by stirring and not due to a physiologically active substance derived from an organism. For example, by conducting the autoclave sterilization treatment with conditions of lower temperature and longer time such as 90°C and 3 hours, it is possible to suppress aggregation or gelation caused by stirring and not due to a physiologically active substance derived from an organism, and sufficiently deactivate a physiologically active substance derived from an organism contaminating the protein.

Further, the surfactant in the present invention may be a negative ionic surfactant, for example, a pure soap content (sodium fatty acid), a pure soap content (potassium fatty acid), sodium alpha-sulfo fatty acid ester, linear alkyl benzene sodium sulfonate, alkylsulfuric acid ester sodium, alkyl ether sulfuric acid ester sodium, sodium alpha-olefin sulfonate and sodium alkylsulfonate. Further, the surfactant may be a nonionic surfactant, for example, Tween (trade name (registered trademark)), Triton (registeredtrademark) X (trade name), Pluronic (trade name (registered trademark)), polyoxyethylene sorbitan monolaurate, polyoxyethylene sorbitan monopalmitate, polyoxyethylene sorbitan monostearate, polyoxyethylene sorbitan monooleate, polyoxyethylene sorbitan trioleate, polyoxyethylene-p-isooctylphenol, a polyethylene oxide/polypropylene oxide block polymer, sucrose fatty acid ester sorbitan fatty acid ester, polyoxyethylene sorbitan fatty acid ester, fatty acid alkanol amide, polyoxyethylene alkyl ether and polyoxyethylene alkylphenyl ether. Further, the surfactant may be an emulsion type antifoam agent obtained by emulsification of the nonionic surfactant with a silicone oil or vegetable oil. Further, the surfactant may be an amphoteric ionic surfactant, for example, alkylamino sodium fatty acid, alkyl betaine and alkyl amine oxide. Further, the surfactant may be a positive ionic surfactant, for example, an alkyltrimethyl ammonium salt and a dialkyltrimethyl ammonium salt. It has been confirmed that addition of these surfactants to a mixture of the sample and the AL reagent when mixed can suppress induction of aggregation or gelation not derived from a physiologically active substance derived from an organism, and thus can suppress more certainly aggregation or gelation caused by stirring and not due to a physiologically active substance derived from an organism.

Further, in the present invention, the final concentration of the predetermined surfactant when the predetermined surfactant is added to the mixture may be 0.0001% or more and 10% or less. This can suppress more effectively aggregation or gelation caused by stirring and not due to a physiologically active substance derived from an organism. Meanwhile, the final concentration is preferably 0.0005% or more and 5% or less, and further preferably 0.005% or more and 0.5% or less.

Meanwhile, addition of the predetermined surfactant, and addition of the predetermined protein that is previously heat-treated (for example, heat treatment-finished albumin), which are of the present invention, may be used in combination in order to suppress aggregation or gelation caused by stirring and not due to a physiologically active substance derived from an organism in the present invention. Herein, as described later, the predetermined protein that is previously heat-treated and the predetermined surfactant may be different in the action mechanism in suppression of the aggregation or gelation caused by stirring and not due to a physiologically active substance derived from an organism. Accordingly, conditions for the predetermined protein that is previously heat-treated to exert the effect may be possibly different from those of the predetermined surfactant. On the other hand, by adding both of the predetermined protein that is previously heat-treated and the predetermined surfactant (the predetermined protein that is previously heat-treated and the predetermined surfactant) to the mixture, it becomes possible to obtain the effect of suppressing aggregation or gelation caused by stirring and not due to a physiologically active substance derived from an organism more certainly in more cases.

Further, the physiologically active substance derived from an organism in the present invention may be endotoxin or β-D-glucan.

In such case, detection or concentration-measurement of endotoxin which is the most typical pyrogen can be carried out more accurately, and it is possible to suppress entry of a transfusion, a medicine for injection, or the blood contaminated with endotoxin into the human body to induce side effects. Similarly, detection or concentration-measurement of β-D-glucan can be carried out more accurately, and it is possible to carry out more accurate screening of infectious diseases due to a variety of fungi including not only fungi which are frequently observed in general clinical practices, such as Staphylococcus, Candida, Aspergillus, and Cryptococcus, but also rare fungi.

Further, the present invention may be a reagent for measuring a physiologically active substance derived from an organism, containing AL which is Amoebocyte lysate, and mixed with the sample containing the predetermined physiologically active substance derived from an organism to produce a mixture, which is used for detection or concentration-measurement of the physiologically active substance in a sample, by detection of aggregation or gelation of a protein due to the physiologically active substance, while stirring the mixture, in which a predetermined protein that is previously heat-treated and/or a predetermined surfactant is added, and aggregation or gelation not derived from the physiologically active substance in the mixture can be suppressed. Further, in such case, the predetermined protein that is previously heat-treated may be at least one kind of albumin, globulin and lysozyme.

Further, in such case, the predetermined protein that is previously heat-treated may be added to the reagent for the measurement such that the final concentration in the mixture is 0.015% or more and 10% or less. Further, the heat treatment may be heating treatment, and the treatment temperature may be 95°C or higher. Further, the treatment time of the heating treatment may be 10 minutes or more. Further, the heat treatment may be autoclave sterilization treatment, the treatment temperature of the autoclave sterilization treatment may be 120°C or higher, and the time of the autoclave sterilization treatment may be 10 minutes or more.

Further, the predetermined surfactant may be a negative ionic surfactant, for example, a pure soap content (sodium fatty acid), a pure soap content (potassium fatty acid), sodium alpha-sulfo fatty acid ester, linear alkyl benzene sodium sulfonate, alkylsulfuric acid ester sodium, alkyl ether sulfuric acid ester sodium, sodium alpha-olefin sulfonate and sodium alkylsulfonate. Further, the predetermined surfactant may be a nonionic surfactant, for example, Tween (trade name (registered trademark)), Triton (registered trademark) X (trade name), Pluronic (trade name (registered trademark)), polyoxyethylene sorbitan monolaurate, polyoxyethylene sorbitan monopalmitate, polyoxyethylene sorbitan monostearate, polyoxyethylene sorbitan monooleate, polyoxyethylene sorbitan trioleate, polyoxyethylene-p-isooctylphenol, a block polymer of polyethylene oxide/polypropylene oxide, sucrose fatty acid ester sorbitan fatty acid ester, polyoxyethylene sorbitan fatty acid ester, fatty acid alkanol amide, polyoxyethylene alkyl ether, polyoxyethylene alkylphenyl ether. Further, the surfactant may be an emulsion type antifoam agent obtained by emulsification of the nonionic surfactant with a silicone oil or vegetable oil. Further, the predetermined surfactant may be an amphoteric ionic surfactant, for example, sodium alkylamino fatty acid, alkyl betaine and alkyl amine oxide. Further, the predetermined surfactant may be a positive ionic surfactant, for example, an alkyltrimethyl ammonium salt and a dialkyltrimethyl ammonium salt.

In such case, the predetermined surfactant may be added to the reagent for the measurement such that the final concentration in the mixture is 0.0001% or more and 10% or less. Further, the physiologically active substance derived from an organism may be endotoxin or β-D-glucan.

Further, the present invention may be an apparatus for measuring a physiologically active substance derived from an organism including: mixture retaining means which retains a mixture of a sample containing a predetermined physiologically active substance derived from an organism and AL, which is Amoebocyte lysate so that light is capable of entering thereinto, and progresses the reaction in the mixture,
stirring means which stirs the mixture in the mixture retaining means,
light emitting means which emits light into the mixture in the mixture retaining means,
light receiving means which receives transmitted light or scattered light of the incident light in the mixture and converts the light into an electrical signal,
determination means which determines the time when the reaction of the physiologically active substance and the AL in the sample initiates from the electrical signal converted in the light receiving means, and
derivation means which derives the presence or concentration of the physiologically active substance in the sample from the predetermined relationship between the reaction initiation time and the concentration of the physiologically active substance, in which the apparatus further includes protein addition means which adds a predetermined protein that is previously heat-treated to the mixture when the mixture is stirred by the stirring means, and/or surfactant addition means which adds a predetermined surfactant to the mixture when the mixture is stirred by the stirring means.

According to this apparatus, it is possible to automatically add the predetermined protein that is previously heat-treated and/or the predetermined surfactant to a mixture of the sample and the AL reagent when the mixture is stirred, and it is possible to suppress more simply aggregation or gelation of a protein that is not derived from the physiologically active substance in the mixture.

Also in this apparatus, the protein added by the protein addition means may be at least one kind of albumin, globulin and lysozyme. Further, in the case where the added protein is albumin, the final concentration of albumin when albumin is added to the mixture may be 0.015% or more and 10% or less. Further, as the heat treatment previously performed for the added protein, for example, autoclave sterilization treatment may be performed. In such case, the temperature of the autoclave sterilization treatment may be 120°C or higher, and the time of the autoclave sterilization treatment may be 10 minutes or more. Further, as the heat treatment previously performed for the added protein, for example, heating treatment using equipment such as an aluminum block heater may be performed. In such case, the temperature of the heat treatment may be 95°C or higher. Further, the heating time in the heating treatment may be 10 minutes or more.

Further, also in this apparatus, the surfactant added by the surfactant addition means may be a negative ionic surfactant, for example, a pure soap content (sodium fatty acid), a pure soap content (potassium fatty acid), sodium alpha-sulfo fatty acid ester, linear alkyl benzene sodium sulfonate, alkylsulfuric acid ester sodium, alkyl ether sulfuric acid ester sodium, sodium alpha-olefin sulfonate or sodium alkylsulfonate. Further, the surfactant may be a nonionic surfactant, for example, Tween (trade name (registered trademark)), Triton (registered trademark) X (trade name), Pluronic (trade name (registered trademark)), polyoxyethylene sorbitan monolaurate, polyoxyethylene sorbitan monopalmitate, polyoxyethylene sorbitan monostearate, polyoxyethylene sorbitan monooleate, polyoxyethylene sorbitan trioleate, polyoxyethylene-p-isooctylphenol, a block polymer of polyethylene oxide/polypropylene oxide, sucrose fatty acid ester sorbitan fatty acid ester, polyoxyethylene sorbitan fatty acid ester, fatty acid alkanol amide, polyoxyethylene alkyl ether or polyoxyethylene alkylphenyl ether. Further, the surfactant may be an emulsion type antifoam agent obtained by emulsification of the nonionic surfactant with a silicone oil or vegetable oil. Further, the surfactant may be an amphoteric ionic surfactant, for example, sodium alkylamino fatty acid, alkyl betaine and alkyl amine oxide. Further, the surfactant may be a positive ionic surfactant, for example, an alkyltrimethyl ammonium salt and a dialkyltrimethyl ammonium salt. Further, the final concentration of the predetermined surfactant when the predetermined surfactant is added to the mixture may be 0.0001% or more and 10% or less. The final concentration of the predetermined surfactant is preferably 0.0005% or more and 5% or less, and further preferably 0.005% or more and 0.5% or less. Furthermore, the physiologically active substance derived from an organism may be endotoxin or β-D-glucan.

Meanwhile, it should be noted that the above-mentioned means for solving the problems of the present invention may be combined to a maximum extent.

### Advantageous Effects of Invention

According to the present invention, in the case where the concentration of a physiologically active substance derived from an organism in a sample is measured by the stirring turbidimetric method, the light scattering method, or the AL-bound bead method, it is possible to suppress aggregation or gelation caused by stirring of the mixture and not derived from the physiologically active substance, whereby to improve the measurement accuracy of the measurement.

### Brief Description of The Drawings

[Fig. 1] A schematic diagram for explaining the gelation process of AL by endotoxin or β-D-glucan, and a detecting method thereof.
[Fig. 2] A diagram illustrating schematic constitution of the apparatus for counting the light scattering particles in Example 1 of the present invention.
[Fig. 3] Agraph illustrating conventional relationship between the concentration of endotoxin in a sample and a gelation detection time, and the calibration curve.
[Fig. 4] A graph illustrating the relationship between the concentration of human serum albumin (HSA) that is added to a mixture of the sample and the AL reagent, and the gelation detection time.
[Fig. 5] A diagram illustrating the calibration curves and the correlation coefficients in the case where HSA was added, and in the case where HSA is not added.
[Fig. 6] A graph illustrating the relationship between the rotation number of the stirring and the gelation detection time.
[Fig. 7] A diagram illustrating schematic constitution of the turbidimetric measuring apparatus in Example 1 of the present invention.
[Fig. 8] A graph illustrating the relationship between the concentration of endotoxin in a sample and the gelation detection time, and the calibration curve in the case where a conventional (non added with a surfactant) AL reagent is used.
[Fig. 9] A graph illustrating the reaction curve in the case where Tween 20 is added to the mixture in Example 2 of the present invention.
[Fig. 10] A graph illustrating the relationship between the concentration of Tween 20 that is added to a mixture of the sample and the AL reagent, and the gelation detection time in Example 2 of the present invention.
[Fig. 11] A graph illustrating the reaction curve in the case where Triton X-100 is added to the mixture in Example 2 of the present invention.
[Fig. 12] A graph illustrating the relationship between the concentration of Triton X-100 that is added to a mixture of the sample and the AL reagent, and the gelation detection time in Example 2 of the present invention.
[Fig. 13] A graph illustrating the relationship between the concentration of endotoxin in a sample and the gelation detection time, and the calibration curve in the case where Tween 20 is added to the mixture in Example 2 of the present invention.
[Fig. 14] A graph illustrating the reaction curves in the case where APB is added to the mixture, in the case where Tween 20 is added to the mixture, and in the case where APB and Tween 20 are added to the mixture.
[Fig. 15] A graph illustrating the reaction curve in the case where APB is added to the mixture, and in the case where Tween 20 is added to the mixture.
[Fig. 16] A diagram illustrating schematic constitution of the apparatus for counting the light scattering particles in Example 3 of the present invention.
[Fig. 17] Adiagram illustrating schematic constitution of the turbidimetric measuring apparatus in Example 3 of the present invention.

### Description of Embodiments

### <Example 1>

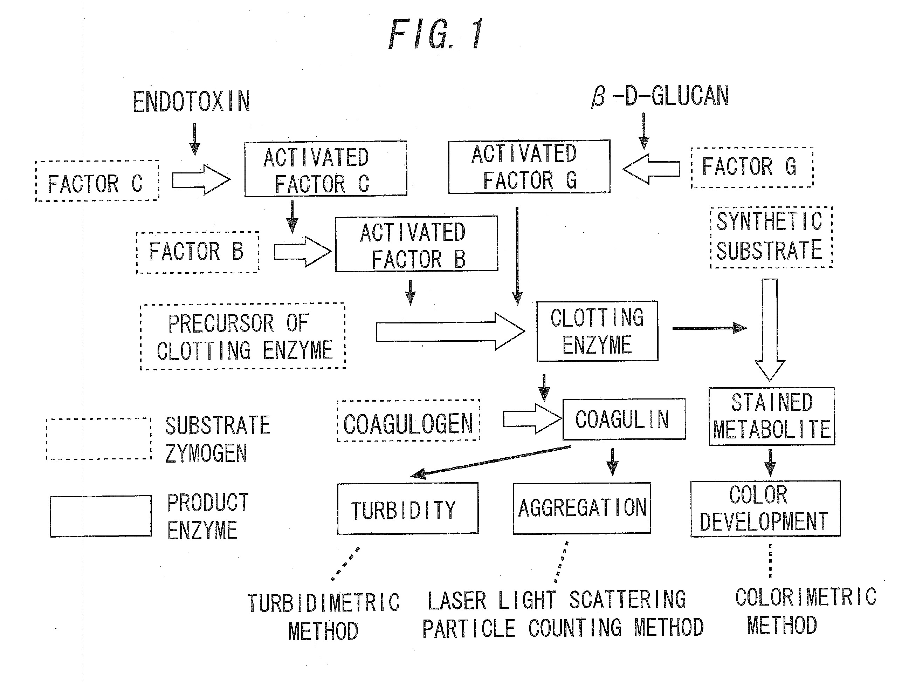

The process of forming a gel by a reaction between AL and endotoxin (hereinafter, also referred to as Limulus reaction.) has been studied well. That is, as illustrated in Fig. 1, when endotoxin is bound to a serine protease, i.e., factor C in AL, the factor C is activated to become activated factor C. The activated factor C hydrolyzes and activates another serine protease, i.e., factor B in AL, and then the factor B is activated to become activated factor B. This activated factor B immediately hydrolyzes a precursor of clotting enzyme in AL to form clotting enzyme, and further the clotting enzyme hydrolyzes a coagulogen in AL to generate coagulin. Thus, the generated coagulin is then associated with each other to further form an insoluble gel, and the whole AL is involved in the formation to turn into a gel.

In addition, similarly, when β-D-glucan is bound to factor G in AL, the factor G is activated to become activated factor G. The activated factor G hydrolyzes a precursor of clotting enzyme in AL to produce clotting enzyme. As a result, similarly to the reaction between endotoxin and the AL, coagulin is generated, and the generated coagulin is associated with each other to further generate an insoluble gel.

The series of reactions as described above are similar to the process of forming a fibrin gel via serine proteases such as Christmas factor or thrombin present in mammals. Such enzyme cascade reactions have a very strong amplification effect because even a very small amount of an activation factor activates the subsequent cascade in a chain reaction. Therefore, according to a method of measuring a predetermined physiologically active substance using AL, it is possible to detect a very small amount (sub-pg/mL order) of the predetermined physiologically active substance.

As a reagent for quantifying endotoxin and β-D-glucan, used is a Limulus reagent using Amoebocyte lysate (AL) as a raw material, and a reagent obtained by adding a synthetic substrate that is hydrolyzed by clotting enzyme to increase any one of coloring intensity, fluorescence intensity or chemical luminescence intensity to a Limulus reagent. Further, a mixed reagent of a recombinant of the factor C (recombinant factor C) in the Limulus reagent and a synthetic substrate thereof (means of coloring, fluorescence and chemical luminescence does not matter) and the like may be used. Furthermore, a mixed reagent of a recombinant of the G factor (recombinant G factor) in the Limulus reagent and a synthetic substrate thereof (means of coloring, fluorescence and chemical luminescence does not matter) and the like may be used.

The physical amount that changes due to the reaction between the predetermined physiologically active substance such as endotoxin and β-D-glucan and the AL may be selected depending on the kind of the reagent. The change of an optical physical amount such as the light transmittance or turbidity, the scattered light intensity, the number of light scattering particles, the absorbance, the fluorescence intensity or the chemical luminescence intensity of the sample may be detected, or the change of a physical amount such as the viscosity of the sample caused by gelation and the electrical conductivity of the sample may be detected. In detection of these physical amounts, optical equipment such as a turbidity meter, an absorption spectrometer, a light scattering spectrometer, a laser light scattering particle counting meter, a fluorescence spectrometer and a photon counter, and a dedicated measurement apparatus by application of them may be used. Further, a viscosity meter, an electrical conductivity meter or a dedicated measurement apparatus by application of them may be used.

Examples of a measurement method which quantifies the predetermined physiologically active substance include the stirring turbidimetric method and the light scattering method, as described above. As illustrated in FIG. 1, in such measurement methods, the aggregate of coagulin formed by the enzyme cascade reactions of the AL is detected with the turbidity of a sample in the former method or with the number of fine gel-particles formed in the system in the latter method, which allows highly sensitive measurement.

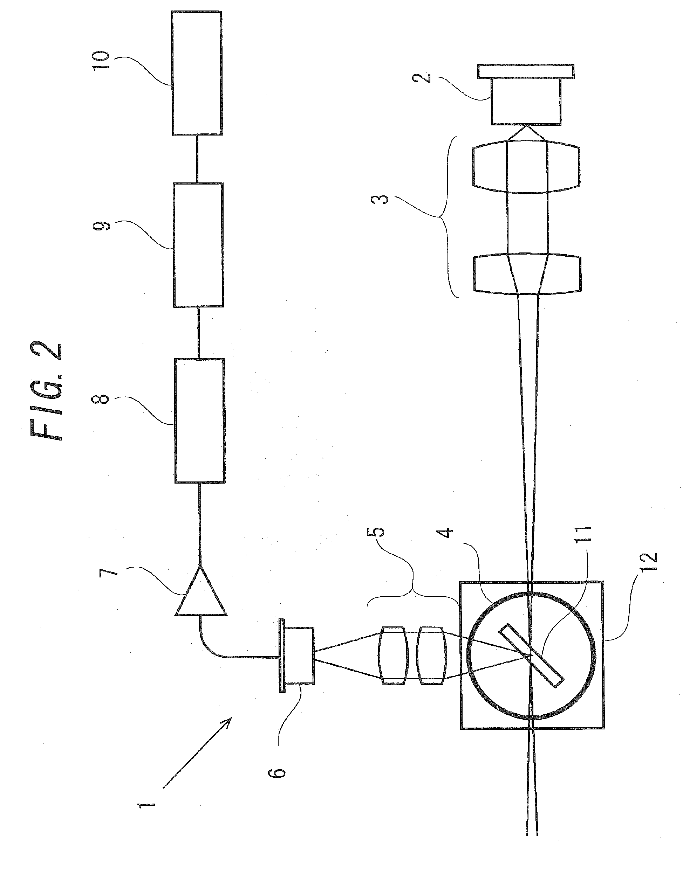

Next, the apparatus for counting the light scattering particles that is used for obtaining scattered light in this Example will be explained. Meanwhile, in the description below, an example is which endotoxin is used as the predetermined physiologically active substance will be explained. However, the description below may be applied to other physiologically active substances such as β-D-glucan and the like. Fig. 2 illustrates the schematic constitution of the apparatus for counting the light scattering particles 1 as an apparatus for measuring endotoxin in this Example. Laser is used as a light source 2 used in the apparatus for counting the light scattering particles 1, but in addition, high-intensity LED or the like may be used as a light source 2. Light emitted from the light source 2 is focused by an optical system for incident light 3 and the focused light enters a sample cell 4. The sample cell 4 retains a mixture of a sample to be measured for endotoxin and an AL reagent. The light which has entered the sample cell 4 is scattered by the particles (the target to be measured such as coagulogen monomers and coagulogen oligomers) in the mixture.

An optical system for outgoing light 5 is arranged laterally to the incident light axis in the sample cell 4. Further, a light receiving element 6 is arranged on an extension of the optical axis of the optical system for outgoing light 5, wherein the light receiving element 6 receives scattered light that has been scattered by particles in the mixture in the sample cell 4 and has been focused by the optical system for outgoing light 5, and which converts the light into an electrical signal. The light receiving element 6 is electrically connected to an amplifier circuit 7 which amplifies the electrical signal obtained by photoelectric conversion in the light receiving element 6, a filter 8 for removing noises from electrical signals amplified by the amplifier circuit 7, a calculation apparatus 9 which calculates the number of gel particles based on the number of peaks of the electrical signals after removal of the noises and further determines a gelation detection time to derive the concentration of endotoxin, and a display 10 which displays the results.

Further, the sample cell 4 is equipped with a magnetic stir bar 11 which rotates by being applied with electromagnetic force from outside for stirring the mixture as a sample, and the sample cell 4 is equipped with a magnetic stirrer 12 in the outside. The stirrers allow adjustment of whether or not to perform stirring, and the stirring rate.

Herein, the sample cell 4 corresponds to the mixture retaining means of this Example. The light source 2 and the optical system for incident light 3 correspond to the light emitting means. The stirring bar 11 and the stirrer 12 correspond to the stirring means. The optical system for outgoing light 5 and the light receiving element 6 correspond to the light receiving means. The calculation apparatus 9 corresponds to the determination means and the derivation means.

In the apparatus for counting the light scattering particles 1, the time to appearance of a coagulin gel particle (gelation detection time = gelation time), which is the final stage of Limulus reaction, is measured, and the concentration of endotoxin in the analyte is calculated using the calibration relationship established between the concentration of endotoxin and the gelation detection time. For example, the gelation detection times when endotoxin solutions of 0.01, 0.001 and 0.0001 EU/mL (1EU/mL ≅ 100 to 200 pg/mL) are measured, are 20 to 30, 40 to 60 and 80 to 150 minutes or so (depending on the kind of the AL reagent or the lot), and a linear calibration curve on a double logarithmic graph is obtained.

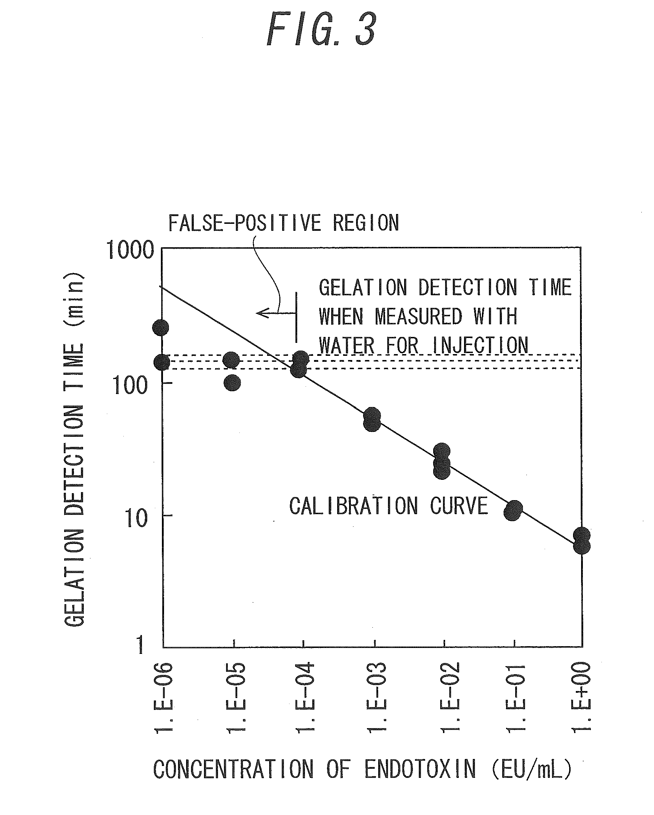

However, in fact, as illustrated in Fig. 3, when an endotoxin solution prepared at further lower concentration than 0.0001 EU/mL or water for injection was measured, the gelation was detected at 80 to 150 minutes or so, and the measurement results in the low concentration region tended to deviate from the calibration curve. Accordingly, if the determination is conducted using the calibration curve as it is, the measurement value of the concentration of endotoxin becomes higher than normal concentration, and false positive determination may be generated. Meanwhile, the AL reagent used in the measurement of Fig. 3 is ES-II Single Test Wako (manufactured by Wako Pure Chemical Industries, Ltd.).

Thus, it becomes clear by investigations of the inventors that this is due to formation of an aggregate (gel particle) not derived from endotoxin, wherein (1) the insoluble aggregate is formed by denatured protein by stimulation of the stirring by the stirring bar 11 and the stirrer 12, or (2) the aggregate is formed by a coagulin gel activated by clotting enzyme by stimulation of the stirring. Accordingly, in order to accurately measure endotoxin in low concentration of 0.0001 EU/mL or less and prevent false positive determination, it is necessary to suppress aggregation not derived from endotoxin.

On the other hand, by intensive study of the inventors, it was found out that aggregation not derived from endotoxin can be suppressed by addition of human serum albumin (HSA). In this Example, an extraction liquid for detecting endotoxin (Wako 293-51601: manufactured by Wako Pure Chemical Industries, Ltd.), i.e., endotoxin-free human serum albumin (HSA) solution (finished with autoclave sterilization) was used, and the effect of suppressing aggregation not derived from endotoxin was investigated by this. Further, as the AL reagent, ES-II Single Test Wako (manufactured by Wako Pure Chemical Industries, Ltd.) was used also in this measurement. Meanwhile, the above-mentioned autoclave sterilization treatment corresponds to the previously performed heat treatment.

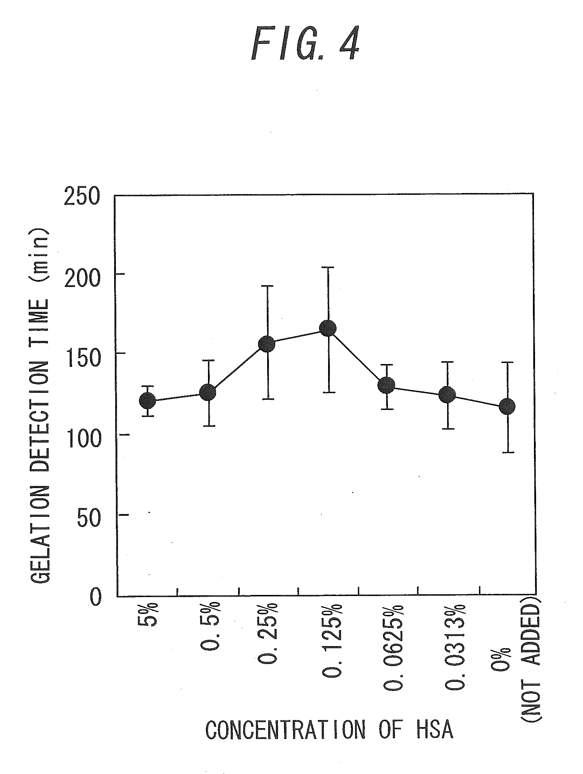

The results of the measurement are illustrated in Fig. 4. The horizontal axis in Fig. 4 is the concentration of HSA in the mixture, and the vertical axis is the gelation detection time. As understood from Fig. 4, in the case where HSA was contained, the gelation detection time tended to be late in comparison to the case where HSA was not contained. Then, particularly in the case where the concentration of HSA was 0.125% to 0.25%, the gelation detection time was remarkably delayed. This suggested that aggregation not derived from endotoxin can be suppressed by addition of a suitable amount of HSA, for example, 0.015% or more and 10% or less of the concentration of HSA.

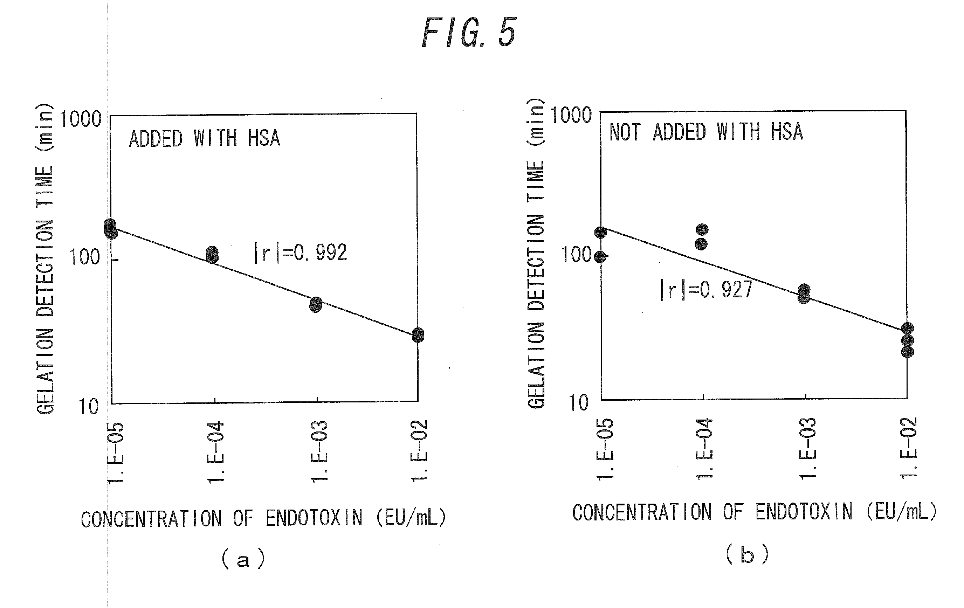

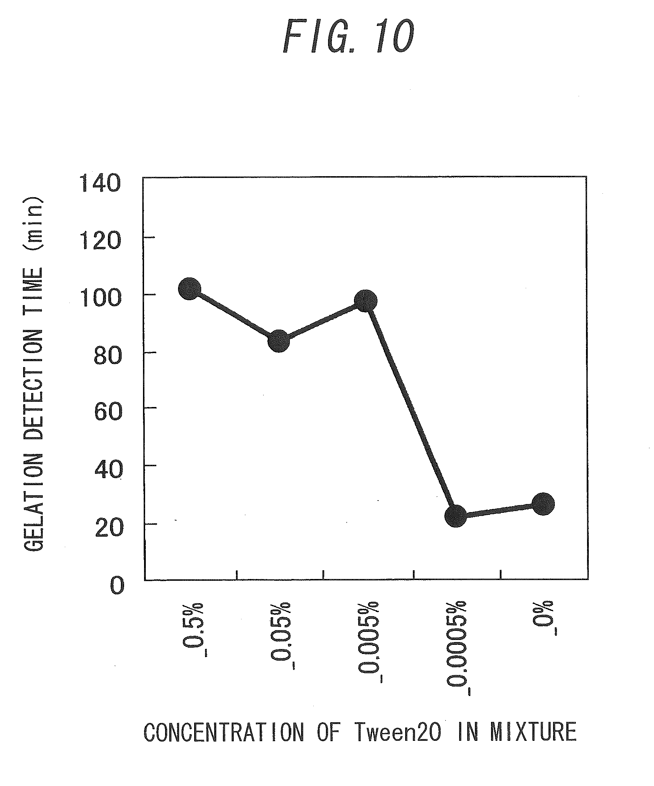

As described above, it was admitted that addition of HSA to the mixture suppressed aggregation not derived from endotoxin, and thus the gelation detection time of the endotoxin solution was measured in the state where HSA was added, and the calibration curve was prepared. The calibration curves in the case where HSA was added, and in the case where HSA was not added, are illustrated in Fig. 5. Fig. 5 (a) illustrates the calibration curve in the case where HSA was added, and Fig. 5 (b) illustrates the calibration curve in the case where HAS was not added. Meanwhile, ES-II Single Test Wako (manufactured by Wako Pure Chemical Industries, Ltd.) was also used as the AL reagent in this measurement. Further, in the measurement in Fig. 5(a), 10 µL of the extraction liquid for detecting endotoxin (Wako 293-51601: manufactured by Wako Pure Chemical Industries, Ltd.) was added along with 200 µL of each concentration of the endotoxin solution to the AL reagent, and the concentration of HSA in the mixture was 0.24%.

As understood from Fig. 5, first, in the case where HSA was added in a range of 0.01 to 0.0001 EU/mL of the concentration of endotoxin, the gelation detection time was not delayed than in the case where HSA was not added. This shows that HSA does not inhibit Limulus reaction. Further, in the case where HSA was added, it was possible to obtain a calibration curve having a good linearity of 0.992 of the correlation coefficient in a range of 1 to 0.00001 EU/mL. This is considered as an effect of suppressing aggregation not derived from endotoxin by addition of HSA. Meanwhile, although a solution of human serum albumin (HSA) (finished with autoclave sterilization) has been used as the extraction liquid for detecting endotoxin, and the effect of suppressing aggregation not derived from endotoxin by this has been investigated in the above, it has been confirmed that a heated human serum albumin preparation for therapeutic use (Albuminar, CSL Behring) also has similar effect. Further, it is considered that globulin or lysozyme, which is a protein having similar property to albumin, also has similar effect when used.

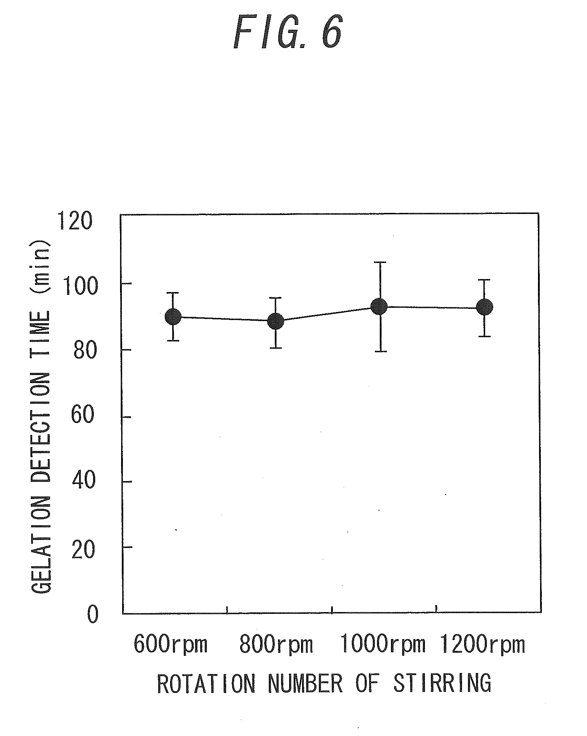

Next, since the aggregation not derived from endotoxin was considered to be caused by stirring of the mixture by the stirring bar 11 and the stirrer 12, lowering of the rotation number of the stirring with respect to the mixture has been investigated. The gelation detection time of water for injection was measured with changing the rotation number, which is usually 1000 rpm, to 600 to 1200 rpm. The results of the measurements are illustrated in Fig. 6. The horizontal axis in Fig. 6 is the rotation number of the stirring bar 11. Further, the vertical axis is the gelation detection time. As understood from the figure, although the rotation number of the stirring bar 11 was changed, the gelation detection time did not become significantly delayed. By this, it was found out that lowering of the stirring rotation number was not expected to have the effect as a measure for suppressing aggregation not derived from endotoxin.

Although the present invention has been explained in the Example above with an example in which the concentration of endotoxin is measured by the light scattering method using the apparatus for counting the light scattering particles 1, it is needless to say that application of the present invention is not limited to the light scattering method. For example, the present invention may be applied to the case where the concentration of endotoxin is measured by the stirring turbidimetric method. Hereinafter, a turbidimetric measuring apparatus used during the stirring turbidimetric method will be explained.

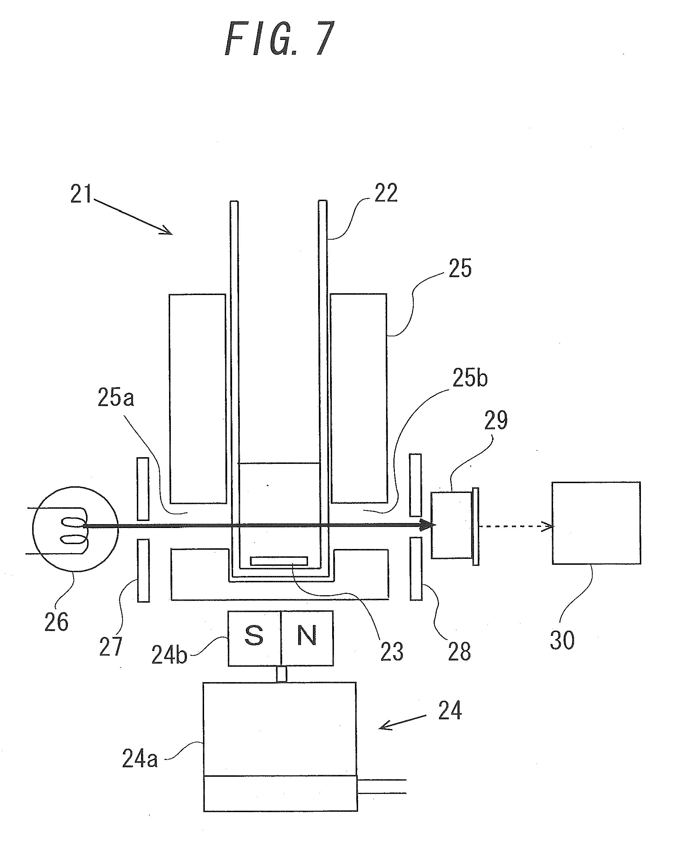

Fig. 7 illustrates the schematic constitution of a turbidimetric measuring apparatus 21 as another example of the apparatus for measuring endotoxin in this Example. With this turbidimetric measuring apparatus 21, endotoxin is measured by the stirring turbidimetric method. Also in the turbidimetric measuring apparatus 21, a sample containing a series of prepared diluted endotoxin is poured into a glass container 22 for measurement as the mixture retaining means (hereinafter, the cuvette). A warmer 25 is disposed so as to surround the cuvette 22. A heating wire not illustrated in the figure is equipped in the inside of this warmer 25, and the cuvette 22 is warmed to about 37°C by electrification of this heating wire. A stainless steel-made stirring bar 23 is equipped in this cuvette 22. This stirring bar 23 rotates in the cuvette 22 by the action of a stirrer 24 installed in the lower part of the cuvette 22. That is, the stirrer 24 is constituted by a motor 24a and a permanent magnet 24b that is disposed on the output axis of the motor 24a. Then, the permanent magnet 24b rotates with electrification of the motor 24a. Since the magnetic field from this permanent magnet 24b rotates, the stainless steel-made stirring bar 23 rotates with the action of the rotating magnetic field. This stirring bar 23 and the stirrer 24 correspond to the stirring means.

Meanwhile, a light source 26 as the light emitting means and a light receiving element 29 as the light receiving means are installed in the turbidimetric measuring apparatus 21. The light emitted from the light source 26 passes through an aperture 27, and then passes through a pore for incidence 25a disposed on the warmer 25 and enters the sample in the cuvette 22. The light transmitted through the sample in the cuvette 22 is emitted from a pore for emission 25b disposed on the warmer 25, passes through an aperture 28 and is applied to the light receiving element 29. The light receiving element 29 outputs photoelectric signal depending on the intensity of the received light. The output of this photoelectric signal is input to a calculation apparatus 30 as the determination means and the derivation means. In the calculation apparatus 30, determination of the reaction initiation time and derivation of the concentration of endotoxin are performed according to a previously loaded program (algorithm). Meanwhile, the turbidimetric measuring apparatus 21 may additionally contain a display apparatus that displays the derived concentration of endotoxin.

Meanwhile, Table 1 shows the gelation detection times in the case where non-heated HSA was added to a mixture of the sample and the AL reagent, and in the case where HSA previously heat-treated with autoclave sterilization treatment (120°C, 15 to 120 minutes) was added. Meanwhile, the concentration of HSA in the mixture was 0.25% in any case. As understood from Table 1, the gelation detection time tended to become delayed in the case where previously heat-treated HSA was added, in comparison to the case where non-heated HSA was added. This showed that that addition of previously heat-treated HSA can suppress more certainly aggregation or gelation caused by stirring and not due to a physiologically active substance derived from an organism.

**[Table 1]**

| | | | | | | |
|---|---|---|---|---|---|---|
| Time of autoclave sterilization | Not heated | 15 minutes | 30 minutes | 60 minutes | 90 minutes | 120 minutes |
| Gelation detection time (min) | 99.9 | 288.0 | 163.7 | 105.2 | 172.1 | 182.7 |

Further, Table 2 shows the gelation detection time in the case where a non-heated human serum albumin preparation (Albuminar) was added to a mixture of the sample and the AL reagent, and in the case where Albuminar previously heat-treated by heating treatment of an aluminum block heater (95°C, 5 to 30 minutes) was added. Also in this case, the albumin concentration in the mixture was 0.25% in any case. As understood from Table 2, also in this case, the gelation detection time tended to become delayed in the case where previously heat-treated Albuminar was added, in comparison to the case where non-heated Albuminar was added. This showed that addition of previously heat-treated Albuminar can suppress more certainly aggregation or gelation caused by stirring and not due to a physiologically active substance derived from an organism.

**[Table 2]**

| | | | | | |
|---|---|---|---|---|---|
| Time of heating treatment | Not heated | 5 minutes | 10 minutes | 20 minutes | 30 minutes |
| Gelation detection time (min) | 108 | 298 | 303 | 172 | 1000 |

### <Example 2>

Next, Example 2 of the present invention will be explained. In Example 2, an example will be explained in which aggregation or gelation caused by stirring and not due to a physiologically active substance derived from an organism is suppressed by addition of a surfactant to a mixture of the sample and the AL reagent, instead of addition of a predetermined protein that is previously heat-treated to a mixture of the sample and the AL reagent. Meanwhile, the apparatus for counting the light scattering particles used in Example 2 is similar to that illustrated in Fig. 2.

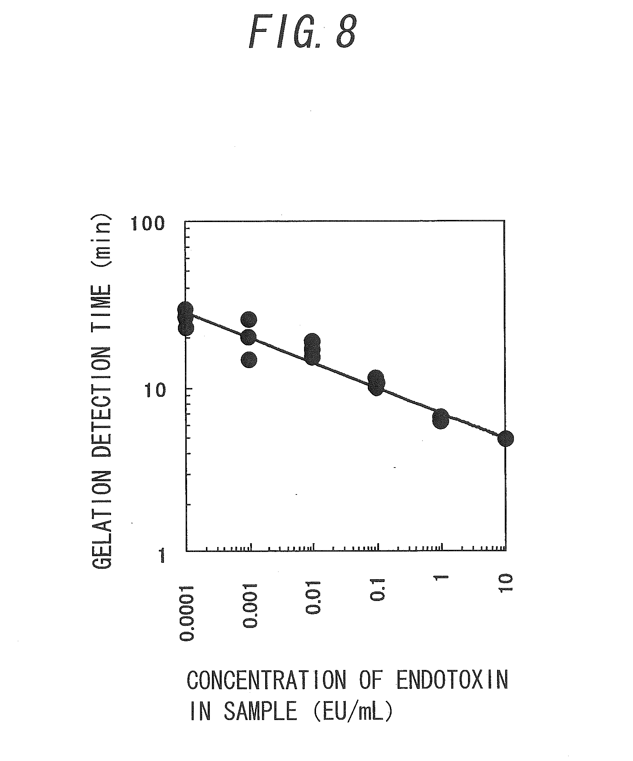

Fig. 8 illustrates the relationship between the concentration of endotoxin and the gelation detection time, and the calibration curve. Meanwhile, in Fig. 8, a mixture obtained by mixing 100 µL of the AL reagent and 100 µL of a sample containing 10 to 0.0001 EU/mL endotoxin was used in the measurement. A calibration relationship was shown in which the gelation detection time definitely became longer as the endotoxin concentration in a sample was lower when the endotoxin concentration in the sample was 10 to 0.01 EU/mL. However, when the endotoxin concentration in the sample was 0.01 EU/mL or less, the gelation detection time was observed to be 14 to 30 minutes or less regardless of the concentration of endotoxin. As a result, it was difficult to accurately measure 0.01 EU/mL or less of endotoxin. In addition, it could not be said that the linearity of the calibration curve is sufficient as the correlation coefficient was 0.974.

This is because the protein is denatured by stimulation of the stirring by the stirring bar 11 and the stirrer 12, and an insoluble aggregate is formed, and this forms an aggregate not derived from endotoxin (gel particle). Accordingly, it is necessary to suppress the aggregation not derived from endotoxin in order to accurately measure a low concentration of endotoxin of 0.01 EU/mL or less. On the other hand, by intensive study of the inventors, it was found out that aggregation not derived from endotoxin can be suppressed by addition of a surfactant such as Tween. Tween 20 and Triton X-100 were used as a surfactant in this Example, and the effect of suppressing aggregation not derived from endotoxin by this was investigated. Meanwhile, any one of Tween 20 and Triton X-100 may be used as it is when the content of endotoxin is small. However, in the case where the content of endotoxin is large, they may be previously autoclave sterilization-treated whereby to inactivate endotoxin.

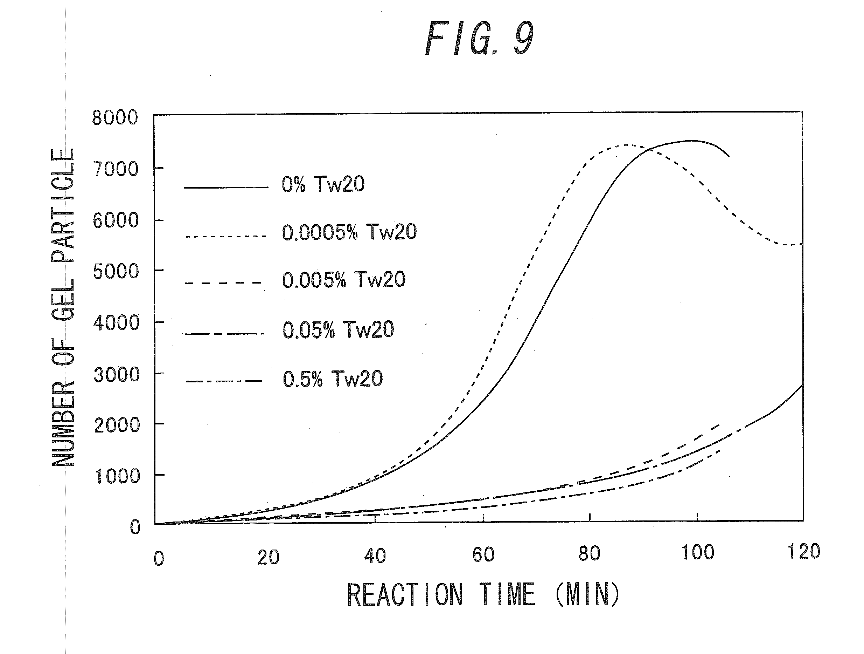

The results of the measurement will be explained below. The reaction curves when a mixture obtained by mixing 100 µL of AL reagent with 100 µL of Tween 20 (0%, 0.001%, 0.01%, 0.1% and 1%) (that is to say, the final concentration of Tween 20 is 0%, 0.0005%, 0.005%, 0.05% and 0.5%) is used for the measurement are illustrated in Fig. 9. The horizontal axis in Fig. 9 represents the reaction time, and the vertical axis represents the number of the gel particles. When Tween 20 is 0% (that is to say, not added) and 0.0005%, the number of the gel particles sharply increases with the time course immediately after initiation of the reaction, reaches the peaks at 80 minutes to 100 minutes or so, and then is reduced. Considered is that this is because the protein is denatured by stimulation of the stirring by the stirring bar 11 and the stirrer 12 immediately after the reaction, and an insoluble aggregate is formed, and this aggregate not derived from endotoxin (gel particle) is measured.

On the other hand, it is understood that when Tween 20 was contained in 0.005% or more, the sharp increase in the number of the gel particles immediately after the reaction initiation is suppressed, and measured number of the gel particles stably increases. From the above, if the final concentration is 0.005% or more and 0.5% or less when the surfactant is Tween or an equivalent substance, an effect of definitely suppressing sharp increase in the number of the gel particles immediately after the reaction initiation can be expected. In consideration of the fact that formation of an aggregate not derived from endotoxin varies depending on the kind of the AL reagent, the maker and the lot, the effect of suppressing sharp increase in the number of the gel particles immediately after the reaction initiation can be expected if the surfactant is generally 0.0001% or more and 10% or less although the degree may vary.

Next, the gelation detection time obtained as the results of the measurement is illustrated in Fig. 10. When Tween 20 was contained in 0.005% or more, the gelation detection time tended to become delayed. This showed that aggregation not derived from endotoxin can be suppressed if Tween 20 is added in a suitable amount.

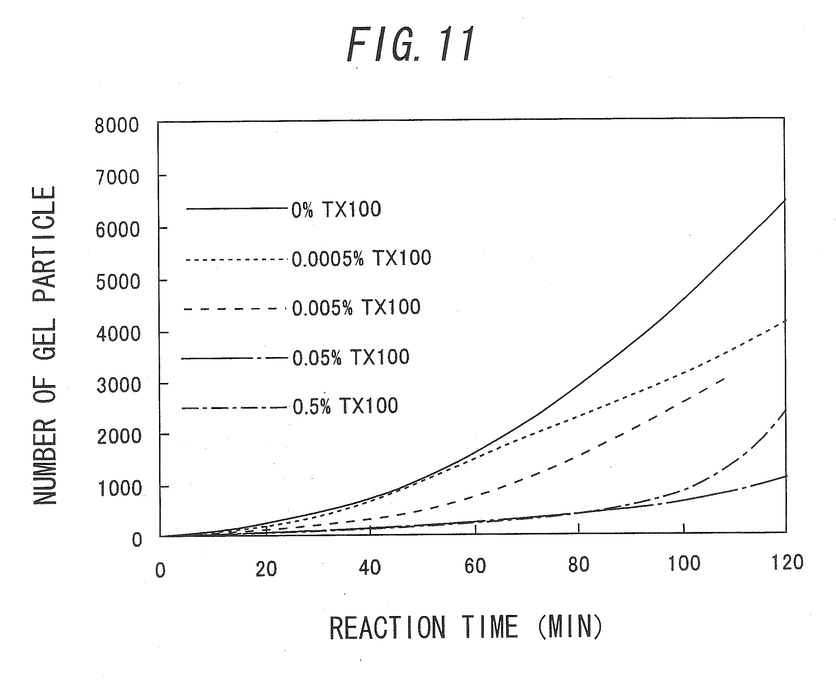

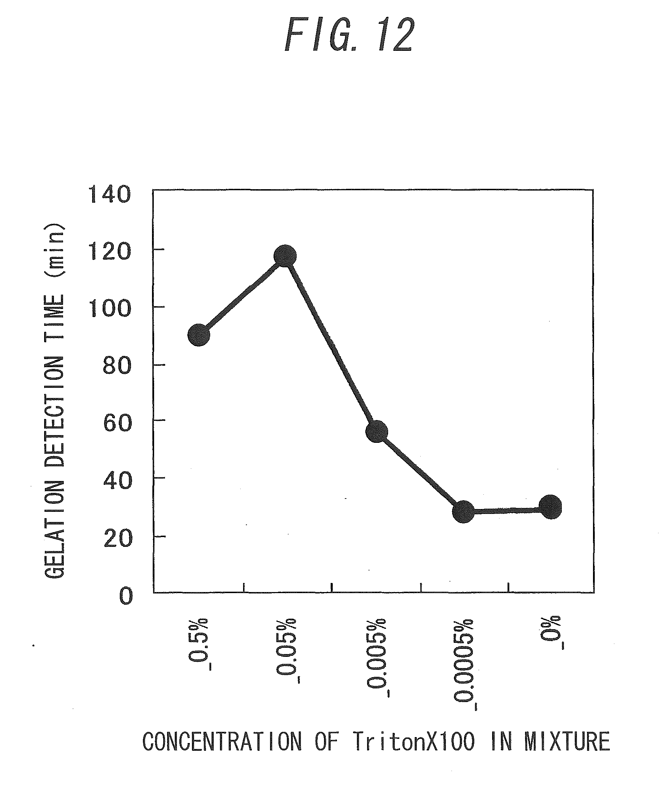

Similarly, the reaction curve when Triton X-100 was used as a surfactant instead of Tween 20 is illustrated in Fig. 11. The horizontal axis in Fig. 11 is the reaction time, and the vertical axis is the number of the gel particles. When Triton X-100 was 0% (that is to say, not added) and 0.0005%, the number of the gel particles sharply increased with the time course immediately after the reaction initiation by formation of the aggregate not derived from endotoxin (gel particle). On the other hand, when Triton X-100 was contained in 0.005% ormore, sharp increase in the number of the gel particles immediately after the reaction initiation was suppressed. The gelation detection time obtained as the result is illustrated in Fig. 12. When Triton X-100 was contained in 0.005% or more, the gelation detection time tended to become delayed.

This showedthat aggregation not derived from endotoxin can be suppressed by addition of Triton X-100 in a suitable amount. From the above, if the final concentration is 0.005% or more and 0.5% or less when the surfactant is Triton X or an equivalent substance, an effect of definitely suppressing sharp increase in the number of the gel particles immediately after the reaction initiation can be expected. In consideration of the fact that formation of an aggregate not derived from endotoxin varies depending on the kind of the AL reagent, the maker and the lot, the effect of suppressing sharp increase in the number of the gel particles immediately after the reaction initiation can be expected if the surfactant is generally 0.0001% or more and 10% or less although the degree may vary.

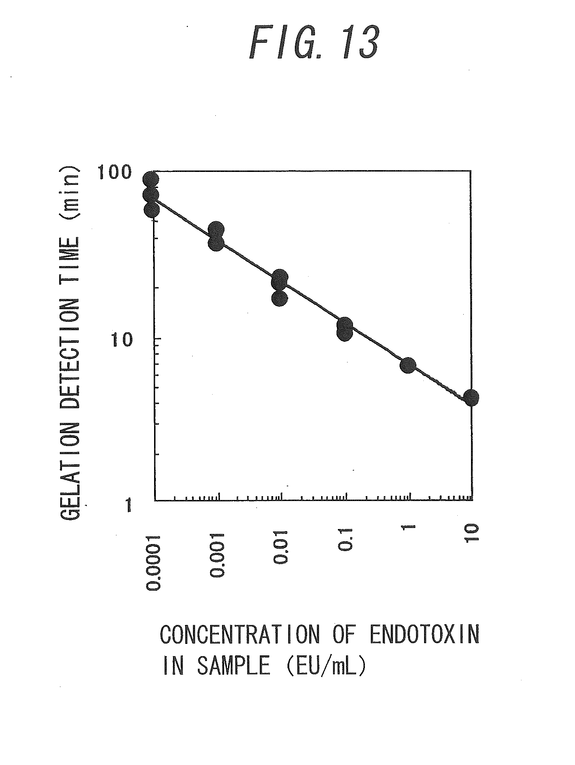

As described above, it was admitted that addition of a surfactant such as Tween 20 and Triton X-100 to the mixture suppressed aggregation not derived from endotoxin, and thus an AL reagent containing Tween 20 in 0.01% was prepared. A mixture obtained by mixing 100 µL of this AL reagent and 100 µL of a sample containing 10 to 0.0001 EU/mL of endotoxin (that is to say, the final concentration of Tween 20 is 0.005%) was used for measurement. The obtained relationship between the concentration of endotoxin and the gelation detection time, and the calibration curve are illustrated in Fig. 13.

As understood from comparison of the calibration curve in the case where Tween 20 was not added as illustrated in Fig. 8 with that of Fig. 13, first, the gelation detection time in the case where Tween 20 was added, did not become delayed than the gelation detection time in the case where Tween 20 was not added, in a range of 10 to 0.01 EU/mL of the endotoxin concentration in the sample. This shows that Tween 20 does not inhibit Limulus reaction. Further, in the case where Tween 20 was added, it was possible to obtain a calibration curve which has good linearity of 0.993 of the correlation coefficient in a range of 10 to 0.0001 EU/mL. This is considered to be an effect of suppressing aggregation not derived from endotoxin by addition of Tween 20.

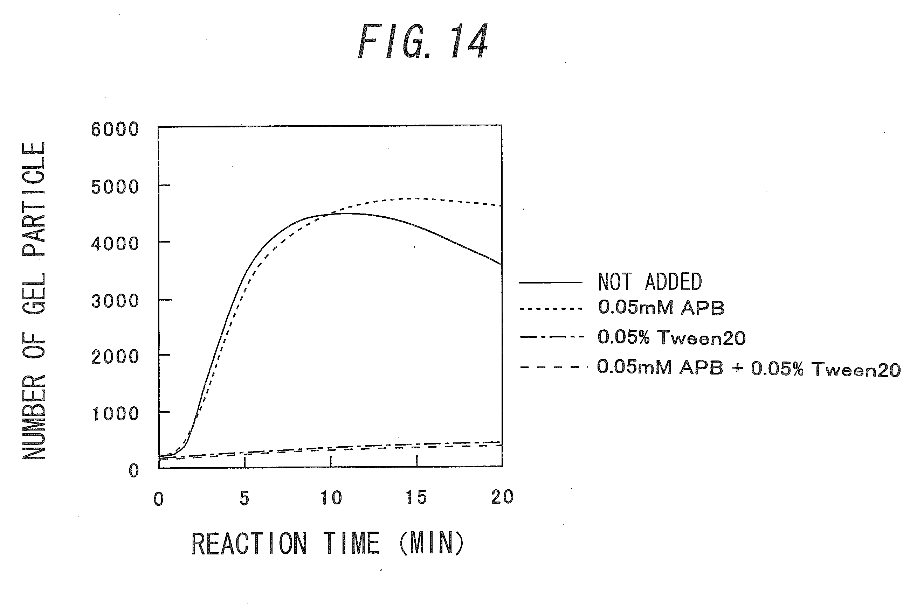

Furthermore, in order to investigate the action mechanism for Tween to suppress aggregation not derived from endotoxin, an experiment for addition of amidinophenyl benzoate (hereinafter, may be described as APB) was performed. APB is one of protease inhibitors, and inhibits activation of clotting enzyme of Limulus reaction. Using another AL reagent, a mixture obtained by mixing 100 µL of the AL reagent and 100 µL of 0.1 mM APB (0. 05 mM APB), and a mixture obtained by mixing 100 µL of the AL reagent and 100 µL of 0.1% Tween 20 (0.05% Tween 20) were used in the measurement, and the reaction curves in such case are illustrated in Fig. 14. Further, the reaction curve when a mixture obtained by mixing 100 µL of the AL reagent containing 0.1 mM APB and 100 µL of 0.1% Tween 20 (0.05 mM APB + 0.05% Tween 20) was previously used in the measurement, is illustrated in Fig. 14.

As understood from Fig. 14, first, in the case where APB was not added, sharp increase in the number of the particles not derived from endotoxin was observed from the initial stage after initiation of the measurement. The greater increase in the number of the particles in comparison to those of Fig. 9 and Fig. 11 is due to difference of the AL reagent. Further, even in the case where APB was added, the increase in the number of the gel particles was not suppressed. This shows that the sharp increase in the number of the gel particles not derived from endotoxin is not those formed by activation of a clotting enzyme due to stimulation of the stirring, and an aggregate formed by aggregation of the proteins to each other is measured. Then, in the case where only Tween 20 was added, and in the case where both of APB and Tween 20 were added, the monotone increase in the number of the gel particles not derived from endotoxin was remarkably suppressed. That is, it was shown that Tween 20 exerts great suppression effect with respect to aggregation of the proteins to each other.

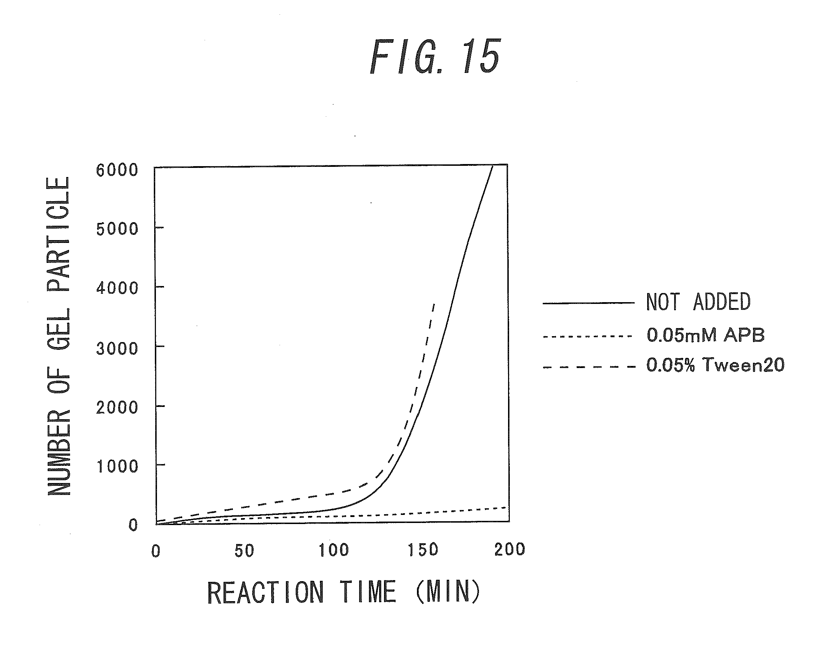

In addition, the reaction curves for another AL reagent used in the case where a mixture obtained by mixing 100 µL of the AL reagent and 100 µL of 0.1 mM APB (0. 05 mM APB), and, a mixture obtained by mixing 100 µL of the AL reagent and 100 µL of 0.1% Tween 20 (0.05% Tween 20) were used in the measurement are illustrated in Fig. 15. As understood from Fig. 15, in the case where APB was not added with use of this AL reagent, sharp increase in the number of the particles from the initial stage after initiation of the measurement was not observed, and at about 100 minutes or delayed, increase in the number of the particles not derived from endotoxin was observed. The manner of increase in the number of the particles in Fig. 15 is different in comparison to those of Fig. 9, Fig. 11 and Fig. 14, and this is because the AL reagent is different. In the case where APB was added, the increase in the number of the gel particles was definitely suppressed. This shows that the increase in the number of the gel particles not derived from endotoxin is caused by activation of a clotting enzyme due to stimulation of the stirring. However, even when Tween 20 was added, the increase in the number of the gel particles not derived from endotoxin was not suppressed. That is, it was shown that Tween 20 has no suppression effect with respect to activation of a clotting enzyme by stimulation of the stirring differently from the heat treatment-finished albumin.

As described above, Tween has different action mechanism from that of the heat treatment-finished albumin that is considered to suppress mainly activation of a clotting enzyme due to stimulation of the stirring, and suppresses aggregation of proteins to each other due to stimulation of the stirring. Accordingly, Tween can be expected to have the effect of suppressing aggregation not derived from endotoxin even with respect to an AL reagent for which the heat treatment-finished albumin cannot be expected to have the effect. Further, Tween can be chemically synthesized differently from albumin that is a natural substance, and thus easily removes mixing in of endotoxin in the initial process. Actually, Tween does not inhibit Limulus reaction (Fig. 13), and delays the gelation detection time by being added (Figs. 9 and 10), which shows that the content of endotoxin of Tween used in this Example is sufficiently low.

Although the present invention has been explained in the Examples above with an example in which the concentration of endotoxin is measured by the light scattering method using the apparatus for counting the light scattering particles 1, application of the present invention is not limited to the light scattering method. For example, the present invention may be applied to the case where the concentration of endotoxin is measured by the stirring turbidimetric method.

Meanwhile, examples using Tween 20 or Triton X-100 as a surfactant to be added to the mixture have been explained in the Examples above. However, as the surfactant, used may be polyoxyethylene sorbitan monolaurate, polyoxyethylene sorbitan monopalmitate, polyoxyethylene sorbitan monostearate, polyoxyethylene sorbitan monooleate, polyoxyethylene sorbitan trioleate, polyoxyethylene-p-isooctylphenol, and the like, which may be under different trade names. Further, equivalent effects can be expected with use of other nonionic surfactants, for example, Pluronic, a block polymer of polyethylene oxide/polypropylene oxide, sucrose fatty acid ester sorbitan fatty acid ester, polyoxyethylene sorbitan fatty acid ester, fatty acid alkanol amide, polyoxyethylene alkyl ether and polyoxyethylene alkylphenyl ether. Further, an emulsion type antifoam agent obtained by emulsification of a silicone oil or vegetable oil with a nonionic surfactant may be used since it is a surfactant and is considered to obtain the effect of suppressing aggregation of proteins to each other although it is not a nonionic surfactant. Further, a negative ionic surfactant, for example, a pure soap content (sodium fatty acid), a pure soap content (potassium fatty acid), sodium alpha-sulfo fatty acid ester, linear alkyl benzene sodium sulfonate, alkylsulfuric acid ester sodium, alkyl ether sulfuric acid ester sodium, sodium alpha-olefin sulfonate, sodium alkylsulfonate may be used. Further, an amphoteric ionic surfactant, for example, sodium alkylamino fatty acid, alkyl betaine and alkyl amine oxide may be used. Further, a positive ionic surfactant, for example, an alkyltrimethyl ammonium salt and a dialkyltrimethyl ammonium salt may be used. Further, the various surfactants may be used in a mixture.

### <Example 3>

Next, Example 3 of the present invention will be explained. The above-mentioned Example 1 has been explained with an example in which the general apparatus for counting the light scattering particles 1 and the turbidimetric measuring apparatus 21 are used in adding HSA to a mixture of the sample and the AL reagent. However, a special apparatus for counting the light scattering particles and a turbidimetric measuring apparatus to automatically add HSA to a mixture of the sample and the AL reagent may be used in order to implement the present invention. In this Example, the apparatus in such case will be explained.

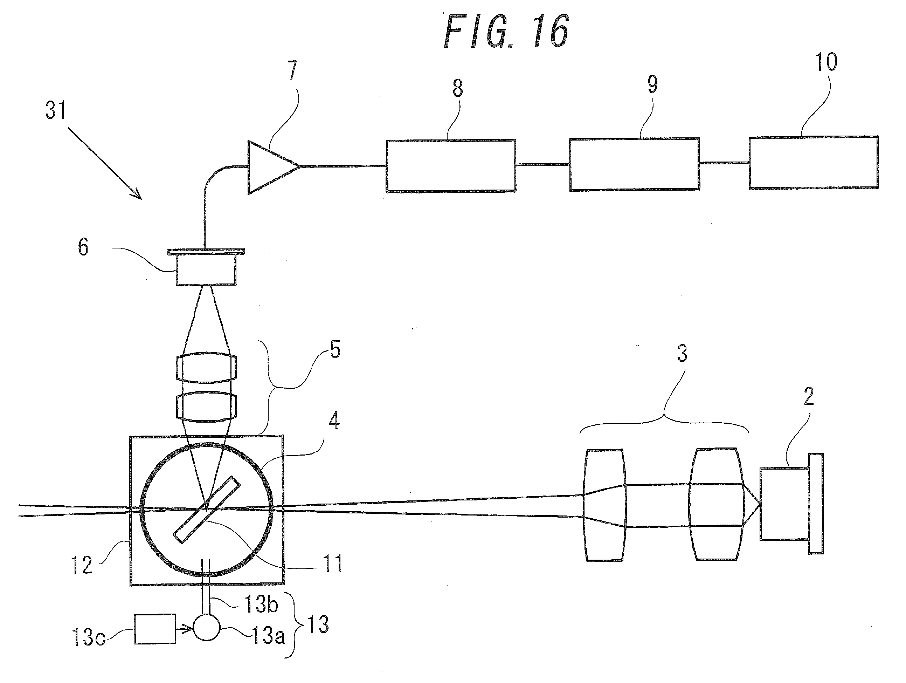

Fig. 16 illustrates an apparatus for counting the light scattering particles 31 that is equipped with an addition apparatus 13 automatically adding a protein such as HSA to a mixture of the sample and the AL reagent. A difference between the apparatus for counting the light scattering particles 31 and the apparatus for counting the light scattering particles 1 explained in Fig. 2 is that the apparatus for counting the light scattering particles 31 has a storage unit 13a and an addition tube 13b. This storage unit 13a stores precisely measured amount of the heat-treated HSA solution before the measurement. Then, when a mixture of the sample and the AL reagent is set up at a measurement position at the time of the measurement, a switch not illustrated in the figure is turned ON, and the storage unit 13a is applied with air pressure, for example, by a small pump 13c. Then, the HSA solution stored in the storage unit 13a is added into the cuvette via the addition tube 13b.

According to this Example, it is possible to lower more simply aggregation that is formed when the mixture is stirred, and not derived from endotoxin, and improve the measurement accuracy of the endotoxin measurement. The addition apparatus 13 corresponds to the protein addition means.

Meanwhile, the addition apparatus 13 in this Example is, of course, not limited to the constitution described above. For example, by a liquid discharge apparatus that can precisely control the discharge amount, the autoclave sterilization-treated HSA solution may be discharged when a mixture of the sample and the AL reagent is set up at the measurement position at the time of the measurement. Alternatively, the autoclave sterilization-treated HSA solution may be previously set up with a dropper sucking the HSA solution in a desired amount, and the HSA solution may be added to the mixture in the glass sample cell 4 by pressing the flexible pump section of the dropper.

Meanwhile, the timing when the heat-treated HSA solution is added to the mixture of the sample and the AL reagent is preferably before the initiation of the stirring by the stirring bar 11. This can suppress more certainly aggregation that is formed when the mixture is stirred, and not derived from endotoxin. However, even at the same time as initiation of the stirring by the stirring bar 11, or after initiation of the stirring, the effect of suppressing aggregation not derived from endotoxin can be obtained by adding the heat-treated HSA solution to the mixture of the sample and the AL reagent.

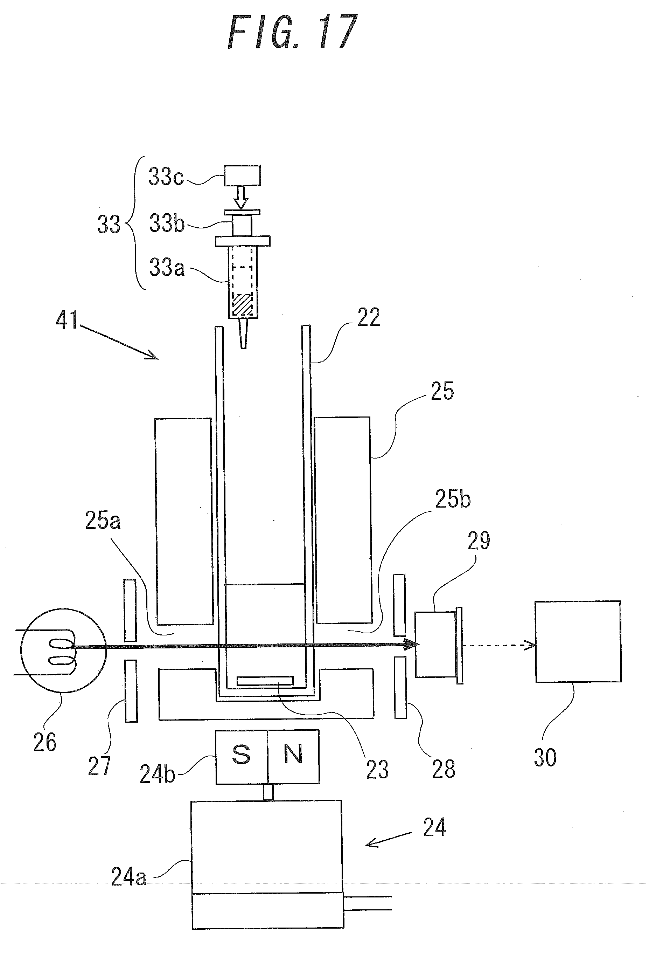

Fig. 17 illustrates a turbidimetric measuring apparatus 41 that is similarly equipped with an addition apparatus 33 automatically adding a protein such as HSA to a mixture of the sample and the AL reagent. A difference between the turbidimetric measuring apparatus 41 and the turbidimetric measuring apparatus 21 explained in Fig. 7 is that the turbidimetric measuring apparatus 41 has an addition apparatus 33 consisting of a syringe 33a, a piston 33b and a piston pressing section 33c. Herein, the syringe 33a retains autoclave sterilization-treated HSA solution sucked in a desired amount. Then, when the cuvette 22 retaining the mixture of the sample and the AL reagent is set up at the measurement position at the time of the measurement, a switch not illustrated in the figure is turned ON, and the piston pressing section 33c presses the piston 33b, and the HSA solution in the syringe 33a is added into the cuvette 22. Also with such turbidimetric measuring apparatus 41, it is possible to lower more simply aggregation that is formed when the mixture is stirred, and not derived from endotoxin, and improve the measurement accuracy of the endotoxin measurement.

Meanwhile, the addition apparatus 33 in this Example is also, of course, not limited to those of the constitution described above, and any apparatus may be used if it has equivalent function.

### <Example 4>

Next, Example 4 of the present invention will be explained. The above-mentioned Example 2 has been explained with an example in which a surfactant such as Tween 20 and Triton X-100 is added to a mixture of the sample and the AL reagent using the general apparatus for counting the light scattering particles 1 and the turbidimetric measuring apparatus 21. However, similarly to those explained in Example 3, a special apparatus for counting the light scattering particles and a turbidimetric measuring apparatus automatically adding a surfactant such as Tween and Triton X to a mixture of the sample and the AL reagent may be used in order to implement the present invention. Meanwhile, the apparatus for counting the light scattering particles in this Example is similar to the apparatus illustrated in Fig. 16. In this Example, by the addition apparatus 13 explained in Example 3, a surfactant such as Tween and Triton X is automatically added to a mixture of the sample and the AL reagent.

The storage unit 13a in this Example stores precisely measured amount of the surfactant such as Tween and Triton X before the measurement. Then, when a mixture of the sample and the AL reagent is set up at a measurement position at the time of the measurement, a switch not illustrated in the figure is turned ON, and the storage unit 13a is applied with air pressure, for example, by the small pump 13c. Then, the surfactant such as Tween and Triton X stored in the storage unit 13a is added into the cuvette via the addition tube 13b.

According to this Example, it is possible to lower more simply aggregation that is formed when the mixture is stirred, and not derived from endotoxin, and improve the measurement accuracy of the endotoxin measurement. The addition apparatus 13 corresponds to the surfactant addition means.

Meanwhile, the addition apparatus 13 in this Example is also, of course, not limited to those of the constitution described above. For example, by a liquid discharge apparatus that can precisely control the discharge amount, the surfactant such as Tween and Triton X may be discharged when a mixture of the sample and the AL reagent is set up at the measurement position at the time of the measurement. Alternatively, the surfactant such as Tween and Triton X may be previously set up with a dropper or pipette sucking the surfactant such as Tween and Triton in a desired amount, and the surfactant such as Tween and Triton X may be added to the mixture in the glass sample cell 4 by pressing the flexible pump section of the dropper or pipette.

Meanwhile, the timing when the surfactant such as Tween and Triton X is added to the mixture of the sample and the AL reagent is preferably before the initiation of the stirring by the stirring bar 11. This can suppress more certainly aggregation that is formed when the mixture is stirred, and not derived from endotoxin. However, even at the same time as initiation of the stirring by the stirring bar 11, or after initiation of the stirring, the effect of suppressing aggregation not derived from endotoxin can be obtained by adding a surfactant such as Tween and Triton X to the mixture of the sample and the AL reagent.

Meanwhile, by the addition apparatus 33 in the turbidimetric measuring apparatus 41 illustrated in Fig. 17, the surfactant such as Tween and Triton X may be automatically added to a mixture of the sample and the AL reagent. Herein, in this Example, a syringe 33a retains a surfactant such as Tween and Triton X sucked in a desired amount. Then, when the cuvette 22 retaining the mixture of the sample and the AL reagent is set up at the measurement position at the time of the measurement, a switch not illustrated in the figure is turned ON, and the piston pressing section 33c presses the piston 33b, and the surfactant such as Tween and Triton X in the syringe 33a is added into the cuvette 22. Also with such turbidimetric measuring apparatus 41, it is possible to lower more simply aggregation that is formed when the mixture is stirred, and not derived from endotoxin, and improve the measurement accuracy of the endotoxin measurement.

Meanwhile, the addition apparatus 33 in this Example is also, of course, not limited to those of the constitution described above, and any apparatus may be used if it has equivalent function.

### Reference Signs List

- 1: Apparatus for counting light scattering particles
- 2: Light source
- 3: Optical system for incident light
- 4: Sample cell
- 5: Optical system for outgoing light
- 6: Light receiving element
- 7: Amplifier circuit
- 8: Filter for removing noise
- 9: Calculation apparatus
- 10: Display
- 11: Stirring bar
- 12: Stirrer
- 13: Addition apparatus
- 21: Turbidimetric measuring apparatus
- 22: Glass container (cuvette)
- 23: Stirring bar
- 24: Stirrer
- 24a: Motor
- 24b: Magnet
- 25: Warmer
- 25a: Pore for incidence
- 25b: Pore for emission
- 26: Light source
- 27: Aperture
- 28: Aperture
- 29: Light receiving element
- 30: Calculation apparatus
- 31: Apparatus for counting light scattering particles equipped with addition apparatus
- 33: Addition apparatus
- 41: Turbidimetric measuring apparatus equipped with addition apparatus

## Claims

1. A method of measuring a physiologically active substance derived from an organism which is used for detection or concentration-measurement of the physiologically active substance in a sample, by detection of aggregation or gelation of a protein due to the reaction of AL, which is Amoebocyte lysate, with the physiologically active substance in a mixture produced by mixing AL and a sample containing the predetermined physiologically active substance derived from an organism while stirring the mixture, **characterized in that**:
by adding a predetermined protein that is previously heat-treated and/or a predetermined surfactant to the mixture during the stirring, aggregation or gelation not derived from the physiologically active substance in the mixture is suppressed.

2. The method of measuring a physiologically active substance derived from an organism according to claim 1, wherein the predetermined protein that is previously heat-treated is at least one kind of albumin, globulin and lysozyme.

3. The method of measuring a physiologically active substance derived from an organism according to claim 1 or 2, wherein the final concentration of the predetermined protein that is previously heat-treated in the case where the predetermined protein that is previously heat-treated is added to the mixture, is 0.015% or more and 10% or less.

4. The method of measuring a physiologically active substance derived from an organism according to any one of claims 1 to 3, wherein the heat treatment is heating treatment, and the treatment temperature thereof is 95°C or higher.

5. The method of measuring a physiologically active substance derived from an organism according to any one of claims 1 to 4, wherein the heat treatment is heating treatment, and the treatment time thereof is 10 minutes or more.

6. The method of measuring a physiologically active substance derived from an organism according to any one of claims 1 to 3, wherein the heat treatment is autoclave sterilization treatment, and the treatment temperature of the autoclave sterilization treatment is 120°C or higher, and the time of the autoclave sterilization treatment is 10 minutes or more.

7. The method of measuring a physiologically active substance derived from an organism according to claim 1, wherein the predetermined surfactant is a nonionic surfactant.

8. The method of measuring a physiologically active substance derived from an organism according to claim 1, wherein the predetermined surfactant contains one kind, or two or more kinds of Tween (registered trademark), Triton (registered trademark) X, Pluronic (registered trademark), polyoxyethylene sorbitan monolaurate, polyoxyethylene sorbitan monopalmitate, polyoxyethylene sorbitan monostearate, polyoxyethylene sorbitan monooleate, polyoxyethylene sorbitan trioleate, polyoxyethylene-p-isooctylphenol, a block polymer of polyethylene oxide/polypropylene oxide, sucrose fatty acid ester sorbitan fatty acid ester, polyoxyethylene sorbitan fatty acid ester, fatty acid alkanol amide, polyoxyethylene alkyl ether and polyoxyethylene alkylphenyl ether.

9. The method of measuring a physiologically active substance derived from an organism according to any one of claims 1, 7 and 8, wherein the final concentration of the predetermined surfactant in the case where the predetermined surfactant is added to the mixture, is 0.0001% or more and 10% or less.

10. The method of measuring a physiologically active substance derived from an organism according to any one of claims 1 to 9, wherein the physiologically active substance derived from an organism is endotoxin or β-D-glucan.

11. A reagent for measuring a physiologically active substance derived from an organism, containing AL which is Amoebocyte lysate, and mixed with the sample containing the predetermined physiologically active substance derived from an organism to produce a mixture, which is used for detection or concentration-measurement of the physiologically active substance in a sample, by detection of aggregation or gelation of a protein due to the physiologically active substance, while stirring the mixture, **characterized in that**:
a predetermined protein that is previously heat-treated and/or a predetermined surfactant is added, and aggregation or gelation not derived from the physiologically active substance in the mixture can be suppressed.

12. The reagent for measuring a physiologically active substance derived from an organism according to claim 11, wherein the predetermined protein that is previously heat-treated is at least one kind of albumin, globulin and lysozyme.

13. The reagent for measuring a physiologically active substance derived from an organism according to claim 11 or 12, wherein the predetermined protein that is previously heat-treated is added such that the final concentration in the mixture is 0.015% or more and 10% or less.

14. The reagent for measuring a physiologically active substance derived from an organism according to any one of claims 11 to 13, wherein the heat treatment is heating treatment, and the treatment temperature thereof is 95°C or higher.

15. The reagent for measuring a physiologically active substance derived from an organism according to any one of claims 11 to 14, wherein the heat treatment is heating treatment, and the treatment time thereof is 10 minutes or more.

16. The reagent for measuring a physiologically active substance derived from an organism according to claim 11 or 12, wherein the heat treatment is autoclave sterilization treatment, and the treatment temperature of the autoclave sterilization treatment is 120°C or higher, and the time of the autoclave sterilization treatment is 10 minutes or more.

17. The reagent for measuring a physiologically active substance derived from an organism according to claim 11, wherein the predetermined surfactant is a nonionic surfactant.

18. The reagent for measuring a physiologically active substance derived from an organism according to claim 11, wherein the predetermined surfactant contains one kind, or two or more kinds of Tween (registered trademark), Triton (registered trademark) X, Pluronic (registered trademark), polyoxyethylene sorbitan monolaurate, polyoxyethylene sorbitan monopalmitate, polyoxyethylene sorbitan monostearate, polyoxyethylene sorbitan monooleate, polyoxyethylene sorbitan trioleate, polyoxyethylene-p-isooctylphenol, a block polymer of polyethylene oxide/polypropylene oxide, sucrose fatty acid ester sorbitan fatty acid ester, polyoxyethylene sorbitan fatty acid ester, fatty acid alkanol amide, polyoxyethylene alkyl ether and polyoxyethylene alkylphenyl ether.

19. The reagent for measuring a physiologically active substance derived from an organism according to any one of claims 11 to 18, wherein the predetermined surfactant is added such that the final concentration in the mixture is 0.0001% or more and 10% or less.

20. The reagent for measuring a physiologically active substance derived from an organism according to any one of claims 11 to 19, wherein the physiologically active substance derived from an organism is endotoxin or β-D-glucan.

21. An apparatus for measuring a physiologically active substance derived from an organism **characterized in that**:
mixture retaining means which retains a mixture of a sample containing a predetermined physiologically active substance derived from an organism and AL, which is Amoebocyte lysate so that light is capable of entering thereinto, and progresses the reaction in the mixture;
stirring means which stirs the mixture in the mixture retaining means;
light emitting means which emits light into the mixture in the mixture retaining means;
light receiving means which receives transmitted light or scattered light of the incident light in the mixture and converts the light into an electrical signal;
determination means which determines the time when the reaction of the physiologically active substance and the AL in the sample initiates from the electrical signal converted in the light receiving means; and
derivation means which derives the presence or concentration of the physiologically active substance in the sample from the relationship between the predetermined reaction initiation time and the concentration of the physiologically active substance,
wherein the apparatus further comprises
protein addition means which adds a predetermined protein that is previously heat-treated to the mixture when the mixture is stirred by the stirringmeans, and/or a surfactant addition means which adds a predetermined surfactant to the mixture when the mixture is stirred by the stirring means.

22. The apparatus for measuring a physiologically active substance derived from an organism according to claim 21, wherein the protein added by the protein addition means is at least one kind of albumin, globulin and lysozyme.

23. The apparatus for measuring a physiologically active substance derived from an organism according to claim 21 or 22, wherein the final concentration of the predetermined protein that is previously heat-treated in the case where the predetermined protein that is previously heat-treated protein is added to the mixture by the protein addition means, is 0.015% or more and 10% or less.

24. The apparatus for measuring a physiologically active substance derived from an organism according to any one of claims 21 to 23, wherein the heat treatment is heating treatment, and the treatment temperature thereof is 95°C or higher.

25. The apparatus for measuring a physiologically active substance derived from an organism according to any one of claims 21 to 24, wherein the heat treatment is heating treatment, and the treatment time thereof is 10 minutes or more.

26. The apparatus for measuring a physiologically active substance derived from an organism according to any one of claims 21 to 23, wherein the heat treatment is autoclave sterilization treatment, and the treatment temperature of the autoclave sterilization treatment is 120°C or higher, and the time of the autoclave sterilization treatment is 10 minutes or more.

27. The apparatus for measuring a physiologically active substance derived from an organism according to claim 21, wherein the surfactant added by the surfactant addition means is a nonionic surfactant.

28. The apparatus for measuring a physiologically active substance derived from an organism according to claim 21, wherein the surfactant added by the surfactant addition means contains one kind, or two or more kinds of Tween (registered trademark), Triton (registered trademark) X, Pluronic (registered trademark), polyoxyethylene sorbitan monolaurate, polyoxyethylene sorbitan monopalmitate, polyoxyethylene sorbitan monostearate, polyoxyethylene sorbitan monooleate, polyoxyethylene sorbitan trioleate, polyoxyethylene-p-isooctylphenol, a block polymer of polyethylene oxide/polypropylene oxide, sucrose fatty acid ester sorbitan fatty acid ester, polyoxyethylene sorbitan fatty acid ester, fatty acid alkanol amide, polyoxyethylene alkyl ether and polyoxyethylene alkylphenyl ether.

29. The apparatus for measuring a physiologically active substance derived from an organism according to any one of claims 21, 27 and 28, wherein the final concentration of the predetermined surfactant in the case where the predetermined surfactant is added to the mixture by the surfactant addition means, is 0.0001% or more and 10% or less.

30. The apparatus for measuring a physiologically active substance derived from an organism according to any one of claims 21 to 29, wherein the physiologically active substance derived from an organism is endotoxin or β-D-glucan.
